(19) Europäisches Patentamt / European Patent Office / Office européen des brevets

(11) **EP 2 828 661 B1**

(12) **FASCICULE DE BREVET EUROPEEN**

(45) Date de publication et mention
de la délivrance du brevet:
**09.03.2016 Bulletin 2016/10**

(21) Numéro de dépôt: **13720436.8**

(22) Date de dépôt: **19.03.2013**

(51) Int Cl.:
*G01N 33/543* (2006.01)   *G01N 33/558* (2006.01)

(86) Numéro de dépôt international:
**PCT/FR2013/050588**

(87) Numéro de publication internationale:
**WO 2013/140089 (26.09.2013 Gazette 2013/39)**

(54) **DISPOSITIF POUR LA DÉTERMINATION D'AU MOINS UN ANALYTE SUSCEPTIBLE D'ÊTRE CONTENU DANS UN ÉCHANTILLON LIQUIDE**

VORRICHTUNG ZUR BESTIMMUNG VON MINDESTENS EINEM ANALYTEN IN EINER FLÜSSIGKEITSPROBE

DEVICE FOR DETERMINING AT LEAST ONE ANALYTE CAPABLE OF BEING CONTAINED IN A LIQUID SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.03.2012   US 201261612564 P
24.10.2012   FR 1260120**

(43) Date de publication de la demande:
**28.01.2015   Bulletin 2015/05**

(73) Titulaire: **Stankov, Milovan
44630 Plesse (FR)**

(72) Inventeur: **Stankov, Milovan
44630 Plesse (FR)**

(74) Mandataire: **Coralis Harle
14-16 Rue Ballu
75009 Paris (FR)**

(56) Documents cités:
**EP-A1- 1 657 550     EP-A1- 1 933 139
WO-A1-98/22800     US-A1- 2007 042 504**

## Description

Dispositif pour la détermination d'au moins un analyte susceptible d'être contenu dans un échantillon liquide

## DOMAINE DE L'INVENTION

La présente invention concerne un dispositif pour la détermination d'au moins un analyte susceptible d'être contenu dans un échantillon liquide.

L'invention a en particulier trait aux dispositifs mettant en oeuvre une technique immunochromatographique par migration latérale.

## ART ANTERIEUR

La plupart des techniques pour la détermination d'analyte(s) ont progressivement évoluées vers des dispositifs de plus en plus simples d'utilisation, permettant le développement de méthodes de diagnostic de routine, rapides et d'un coût modéré.

Cette évolution a été particulièrement sensible dans le domaine médical, avec l'émergence du diagnostic par « point of care » ou « home testing », dans lequel un diagnostic est directement réalisé près du lit du patient ou à son domicile, sans qu'il soit nécessaire de faire appel aux techniques automatisées de laboratoire d'analyse.

Les immuno-essais font partie des technologies utilisées pour ce type de diagnostic rapide.

Ces immuno-essais regroupent les dispositifs et méthodes de diagnostic basés sur des réactions de liaisons par affinité entre membres de paires de liaisons spécifiques.

Globalement, ces immuno-essais sont divisés en deux grandes approches bien connues.

Dans l'approche dite « compétition », l'analyte recherché et un réactif de détection marqué sont en compétition pour se lier spécifiquement à un réactif de capture. La présence ou l'absence de l'analyte recherché dans l'échantillon sont mesurées, respectivement, par l'absence ou par la présence d'un signal visible (ou mesurable) au niveau du réactif de capture.

Dans l'approche dite « sandwich », un réactif de détection marqué se lie à l'analyte recherché, ce dernier étant immobilisé sur le support solide par l'intermédiaire du réactif de capture. La présence ou l'absence de l'analyte dans l'échantillon liquide sont mesurées, respectivement, par la présence ou par l'absence d'un signal visible (ou mesurable) au niveau du réactif de capture.

Parmi ces immuno-essais, il est connu des dispositifs dits « immunochromatographiques » qui se prêtent particulièrement à certains analytes.

L'immunochromatographie consiste en une méthode de diagnostic en phase solide, utilisant la chimie sèche et la migration latérale sur une membrane inerte.

Ce type d'immuno-essai met en oeuvre un support poreux sous forme de bandelette, dans et/ou sur lequel sont intégrés sous forme sèche les réactifs nécessaires à la réalisation du test.

Le support poreux est traité de façon à ce qu'une réaction de reconnaissance entre partenaires de liaison spécifique (généralement antigène / anticorps) se produise au niveau d'une zone de capture et puisse être révélée à ce niveau.

En pratique, lorsque l'échantillon liquide à analyser est déposé sur le support, celui-ci migre le long de la membrane par un phénomène de capillarité. L'analyte recherché est généralement capturé par un réactif de capture spécifique immobilisé sur une zone déterminée de la membrane.

La réaction est révélée par un réactif de détection spécifique marqué, selon le principe de la méthode sandwich.

Il est également possible d'utiliser une réaction de type compétition. On utilise dans ce cas généralement un réactif de détection consistant en un analyte marqué qui est compétiteur de l'analyte recherché pour la liaison spécifique avec le réactif de capture immobilisé.

Ces dispositifs immunochromatographiques sont généralement adaptés à un usage unique et domestique. En effet, ils sont d'un usage facile et rapide, ne nécessitant que très peu de manipulations puisque tous les réactifs sont intégrés ou compris dans le dispositif.

Cependant, dans certaines situations particulières, ces dispositifs immunochromatographiques sont susceptibles de donner des résultats qui ne sont pas totalement fiables.

D'une part, lors de la détermination d'un analyte par un test au format « sandwich », on observe pour certains analytes un effet dit « crochet » (désigné encore par « hook effect » en anglais).

L'effet crochet est un effet indésirable bien connu dans les tests immunologiques. Il se produit lorsque l'analyte est présent dans l'échantillon à une concentration très élevée.

L'effet crochet peut alors conduire à des faux négatifs, amenant à conclure de façon aberrante à l'absence ou à une faible concentration de l'analyte dans l'échantillon.

Les analytes présentant un effet crochet lors de dosages immunologiques de type sandwich génèrent des courbes signal/concentration du type courbe de Gauss.

Pour un signal donné, deux concentrations sont alors possibles pour l'analyte recherché, l'une faible et l'autre élevée, lors de la lecture du résultat à un temps défini.

Un test par compétition permet l'obtention de deux signaux différents pour deux concentrations respectivement différentes de l'analyte à doser.

Mais, les tests par compétition montrent également très rapidement leurs limites, car il y a une extinction du signal à des concentrations relativement peu élevées de l'analyte d'intérêt.

Une solution communément adoptée pour remédier aux inconvénients de ces tests sandwich et compétition consiste à doser l'analyte à partir d'une gamme de dilution de l'échantillon liquide.

Mais l'utilisation d'une telle gamme de dilution ne convient pas un usage domestique. En outre, l'utilisation d'une gamme de dilution de l'échantillon nécessite des manipulations supplémentaires et une consommation accrue de dispositifs de test à usage unique, puisque chaque échantillon est testé aux différentes dilutions.

Une solution à cet effet est par exemple décrite dans le document WO 2007/023372, concernant un dispositif de détermination d'un analyte dans un échantillon liquide, comportant un moyen de diffusion capillaire sur lequel sont matérialisés :

a) une zone de dépôt ou réception de l'échantillon ;
b) une zone amont de libération comprenant un réactif de détection spécifique de l'analyte conjugué à un marqueur visible et/ou mesurable, libre de migrer par diffusion capillaire à l'état humide dans le moyen de diffusion capillaire ; et
c) deux zones aval de capture comprenant, successivement dans la direction de diffusion capillaire, d'une part, un réactif de capture spécifique de l'analyte et, d'autre part, l'analyte, ou un analogue de l'analyte, immobilisé.

Le réactif de détection et le réactif de capture, spécifiques de l'analyte, permettent la détermination de l'analyte dans l'échantillon liquide par un test sandwich ; ensuite ce même réactif de détection spécifique de l'analyte et l'analyte, ou l'analogue de l'analyte, immobilisé, permettent la détermination de l'analyte d'intérêt dans l'échantillon liquide par un test de compétition.

Dans un tel dispositif, le réactif de détection est déposé en excès au niveau d'une unique zone de libération qui est ménagée en amont des zones de capture complémentaires.

Malgré son intérêt, les inventeurs ont constaté que cet excès de réactif de détection en amont des zones de capture n'est pas satisfaisant, en ce sens qu'il est fréquent d'observer des réactions non spécifiques, un bruit de fond prononcé et des problèmes de migration.

D'autre part, certains dispositifs immunochromatographiques sont structurés pour la détermination simultanée de plusieurs analytes.

Un tel dispositif immunochromatographique, par exemple décrit dans le document EP-1 657 550, comporte avantageusement un moyen de diffusion capillaire sur lequel sont matérialisés :

a) une zone de dépôt ou réception de l'échantillon ;
b) une zone amont de libération comprenant un mélange de réactifs de détection spécifiques chacun de l'un des analytes, conjugués à un marqueur visible et/ou mesurable, libres de migrer par diffusion capillaire à l'état humide dans le moyen de diffusion capillaire ; et
c) des zones aval de capture comprenant chacune un réactif de capture qui est spécifique de l'un des analytes, lesdites zones aval de capture étant répartis successivement selon le sens de migration.

Là encore, les réactifs de détection sont déposés au niveau de la zone de libération unique qui est matérialisée en amont des zones de capture, tenant compte du sens de migration capillaire.

Or, les inventeurs ont là encore constaté que ce mélange des réactifs de détection, en amont des différentes zones de capture, n'est pas satisfaisant à cause de réactions croisées entre les différents réactifs de détection mais aussi d'inhibition d'activité spécifique mutuelle entre les réactifs.

Les dispositifs immunochromatographiques comprenant une zone de libération du ou des réactifs de détection, qui est ménagée en amont d'une pluralité de zones de capture, ne sont ainsi pas satisfaisants.

Il existe par conséquent un besoin pour de nouveaux dispositifs de détermination à diffusion capillaire, aussi bien pour la détermination d'un unique analyte que pour la détermination de plusieurs analytes, permettant de pallier aux problèmes générés notamment par l'excès et/ou le mélange de réactif(s) de détection rapporté(s) en amont d'une pluralité de zones de capture successives.

## RESUME DE L'INVENTION

La présente invention concerne ainsi un dispositif pour la détermination de la présence et/ou de la quantité d'au moins un analyte susceptible d'être contenu dans un échantillon liquide, comportant un moyen de diffusion capillaire sur lequel ledit échantillon liquide est destiné à migrer latéralement selon une direction et un sens de migration capillaire, tel que défini dans les revendications.

Différentes zones sont matérialisées sur ce moyen de diffusion capillaire, dans ledit sens de migration capillaire amont vers aval, à savoir au moins :

- une zone de dépôt de l'échantillon liquide,
- une zone amont de libération qui comprend au moins un réactif de détection conjugué avec un marqueur visible et/ou mesurable, ledit réactif de détection étant apte à se déplacer en conséquence de la migration de l'échantillon liquide dans ledit moyen de diffusion capillaire, et
- au moins deux zones de capture qui comprennent chacune au moins un réactif de capture, immobilisé sur ledit moyen de diffusion capillaire.

Ce dispositif comporte encore au moins une zone aval de libération qui est matérialisée sur ledit moyen de diffusion capillaire et qui se situe en aval de l'une au moins desdites zones de capture.

Cette zone aval de libération comprend également au moins un réactif de détection conjugué à un marqueur visible et/ou mesurable, ledit réactif de détection étant apte à se déplacer en conséquence de la migration de l'échantillon liquide dans le moyen de diffusion capillaire.

Le réactif de détection d'une zone de libération et/ou le réactif de capture de la ou des zones de capture complémentaires, situées directement en aval de ladite zone de libération, sont alors aptes à se lier spécifiquement avec ledit analyte et/ou à se lier spécifiquement l'un avec l'autre, pour former un complexe permettant la détermination dudit analyte dans ledit échantillon liquide au niveau de ladite ou desdites zones de capture complémentaires.

La ou les zones aval de libération sont chacune intercalées entre deux zones de capture.

De manière générale, un tel agencement a en particulier l'intérêt de permettre une adaptation du réactif de détection d'une zone de libération, en quantité et/ou en spécificité, en fonction du réactif de capture constituant la ou les zones de capture situées directement en aval de ladite zone de libération. D'autres caractéristiques avantageuses, pouvant être prises en combinaison ou indépendamment les unes des autres, sont développées ci-dessous :

- un groupe amont de zones comprend une zone de capture amont située directement en aval de la zone de libération amont, avantageusement adapté pour un test au format sandwich, lequel groupe amont de zones est lui-même suivi d'un ou plusieurs groupes de zones comprenant chacun une zone de libération et une ou plusieurs zones de capture complémentaires ; cette caractéristique permet d'optimiser la concentration et la spécificité du ou des groupes de zones aval en fonction des seuils de détection du ou des analytes à déterminer.

Pour la détermination d'un unique analyte, le ou les réactifs de détection des zones de libération et/ou le ou les réactifs de capture des zones de capture sont avantageusement aptes à se lier spécifiquement avec ledit analyte.

Dans ce cas, les zones de libération comportent de préférence un ou des réactifs de détection identiques entre elles, et les zones de captures comportent de préférence un ou des réactifs de capture identiques entre elles.

Encore dans ce cas, le moyen de diffusion capillaire comporte avantageusement :

- la zone amont de libération,
- une première zone de capture ou deux premières zones de capture, complémentaire(s) de ladite zone amont de libération, puis
  soit
- une zone aval de libération, et
- au moins deux secondes zones de capture, complémentaires de ladite zone aval de libération,
  soit au moins deux couples successifs de zones comprenant chacun :
- une zone de libération, et
- au moins une zone de capture, complémentaire de ladite zone de libération associée.

Pour la détection d'au moins deux analytes, le réactifs de détection d'une zone de libération et/ou le réactif de capture de la ou des zones de capture complémentaires, immédiatement en aval de ladite zone de libération, sont avantageusement aptes à se lier spécifiquement avec l'un desdits analytes.

Selon encore d'autres caractéristiques avantageuses, pouvant être prises en combinaison ou indépendamment les unes des autres :

- la ou les zones aval de libération matérialisées sur le moyen de diffusion capillaire, chacune intercalée entre deux zones de capture, sont au nombre de 1 à 4, par exemple au nombre de 2 pour un format de support capillaire standard de 25 mm ;
- la ou les zones de capture matérialisées sur le moyen de diffusion capillaire, complémentaires de l'une des zones de libération située directement en amont, sont au nombre de 1 à 10, préférablement au nombre de 5 pour un format de support capillaire standard de 25mm ;
- le réactif de détection de l'une au moins des zones de libération et le réactif de capture de la ou de l'une des zones de capture complémentaires sont aptes à se lier spécifiquement avec l'analyte ou l'un au moins des analytes, pour constituer un test au format sandwich ;
- le réactif de détection de l'une au moins des zones de libération et le réactif de capture de la ou de l'une au moins des zones de capture complémentaires consistent, pour l'un, en un analogue de l'analyte à déterminer et, pour l'autre, en un réactif apte à se lier spécifiquement avec ledit analyte ou avec ledit analogue de l'analyte, pour constituer un test au format compétition ;
- la zone de dépôt de l'échantillon liquide est (i) confondue avec la zone amont de libération ou (ii) située en amont de la zone amont de libération.

La présente invention concerne encore un procédé pour la détermination quantitative d'un analyte dans un échantillon liquide déposé sur un dispositif de détermination ; ce procédé comprend les étapes successives suivantes :

- une étape de mesure de l'intensité du signal au niveau de chaque zone de capture,
- dans chaque groupe de zones, une étape d'additionnement desdites intensités mesurées,
- une étape de calcul d'un ratio d'intensité correspondant aux intensités additionnées pour un groupe de zones par rapport aux intensités additionnées pour un autre groupe de zones, et
- une étape d'association dudit ratio d'intensité calculé à une valeur quantitative de l'analyte dans ledit échantillon liquide tenant compte d'une courbe étalon correspondant au ratio d'intensité calculé en fonction d'une quantité d'analyte dans ledit échantillon liquide.

## FIGURES

La présente invention sera encore illustrée, sans aucunement être limitée, par la description suivante de différents dispositifs conformes à l'invention, cela en relation avec les dessins annexés dans lesquels :

- la figure 1 représente, de manière schématique, un premier dispositif selon l'invention pour la détermination d'un unique analyte, comprenant deux groupes de zones composés chacun d'une zone de libération et d'une zone de capture ;
- la figure 2 représente, encore de manière schématique, un deuxième dispositif selon l'invention pour la détermination d'un unique analyte, comprenant un premier groupe de zones composé d'une zone de libération et d'une zone de capture, puis un second groupe de zones composé d'une zone de libération suivie de plusieurs zones de capture ;
- la figure 3 illustre, également de manière schématique, un troisième dispositif selon l'invention pour la détermination d'un unique analyte, comprenant plusieurs groupes de zones composés chacun d'une zone de libération et d'une zone de capture ;
- la figure 4 montre, encore de manière schématique, un quatrième dispositif selon l'invention pour la détermination de deux analytes, comprenant deux groupes de zones composés chacun d'une zone de libération et d'une zone de capture, dédiés chacun à l'un desdits analytes ;
- la figure 5 représente, toujours de manière schématique, un cinquième dispositif selon l'invention pour la détermination de plusieurs analytes, comprenant plusieurs groupes de zones composés chacun d'une zone de libération et de deux zones de capture, dédiés chacun à l'un des analytes.

## DESCRIPTION DETAILLEE DE L'INVENTION

La présente invention concerne ainsi un dispositif pour la détermination d'au moins un analyte susceptible d'être contenu dans un échantillon liquide.

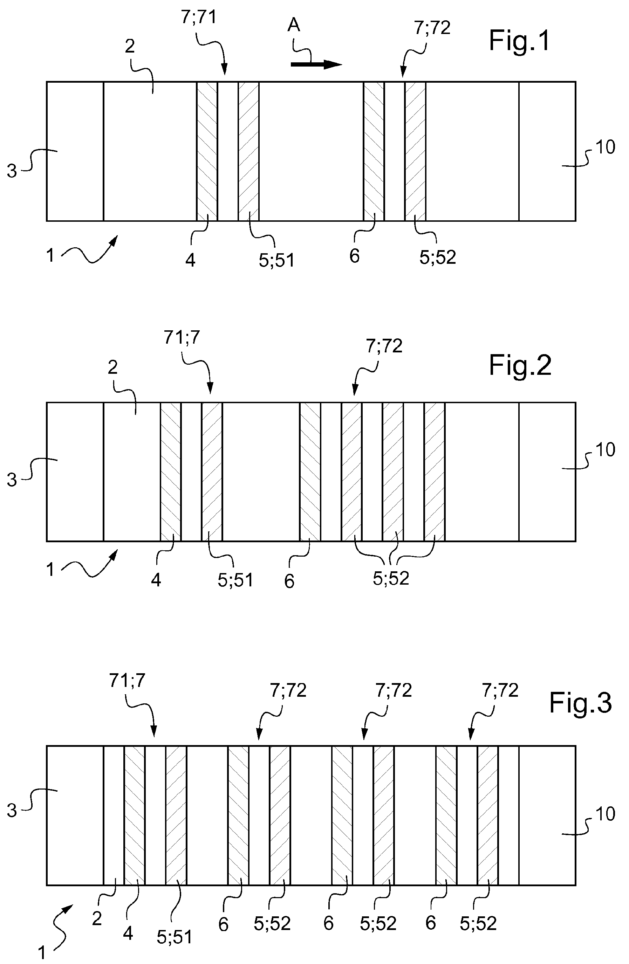

La structure générale de ce dispositif selon l'invention est illustrée très schématiquement au travers des divers modes de réalisation représentés sur les figures 1 à 5 précitées.

De manière générale, les repères numériques employés sont conservés pour désigner les éléments structurels identiques ou similaires lors de la description des différents modes de réalisation.

Tel que représenté sur les figures 1 à 5, chaque dispositif 1 comporte un moyen de diffusion capillaire 2 sur lequel ledit échantillon liquide (non représenté) est destiné être déposé puis à migrer latéralement selon une direction et un sens de migration capillaire amont vers aval.

La direction et le sens de migration sont illustrés schématiquement par la flèche désignée par le repère A sur la figure 1.

De manière générale, les notions de « amont » et « aval » se réfèrent à cette direction et à ce sens de migration de l'échantillon liquide sur la longueur du moyen de diffusion capillaire 2.

Différentes zones successives sont matérialisées sur ce moyen de diffusion capillaire 2, dans ledit sens de migration capillaire amont vers aval A, à savoir au moins :

- une zone 3 pour le dépôt de l'échantillon liquide,
- une zone amont de libération 4 qui comprend au moins un réactif de détection conjugué avec un marqueur visible et/ou mesurable, ledit réactif de détection étant apte à se déplacer en conséquence de la migration de l'échantillon liquide dans ledit moyen de diffusion capillaire 2, et
- au moins deux zones de capture 5 qui comprennent chacune au moins un réactif de capture, immobilisé sur ledit moyen de diffusion capillaire.

Selon l'invention, ce dispositif 1 comporte encore au moins une zone supplémentaire de libération 6, qui est matérialisée sur ledit moyen de diffusion capillaire 2 et qui se situe en aval de l'une au moins desdites zones de capture 5.

Cette zone supplémentaire de libération 6, aval, comprend également au moins un réactif de détection conjugué à un marqueur visible et/ou mesurable, ledit réactif de détection étant apte à se déplacer en conséquence de la migration de l'échantillon liquide dans le moyen de diffusion capillaire.

Le milieu de diffusion capillaire 2 comporte ainsi plusieurs groupes successifs de zones 7 qui se composent chacun d'au moins deux zones successives :

- une zone de libération 4 ou 6, en amont, et
- une ou plusieurs zones de capture 5, dite « complémentaire », situées directement en aval de ladite zone de libération 4, 6 associée.

Le réactif de détection d'une zone de libération 4, 6 et/ou le réactif de capture de la ou des zones de capture complémentaires 5 sont choisis pour se lier spécifiquement avec ledit analyte et/ou à se lier spécifiquement l'un avec l'autre.

Cette approche assure la formation de complexe(s) permettant la détermination dudit analyte dans ledit échantillon liquide au niveau de ladite ou desdites zones de capture complémentaires 5.

Les réactifs de détection et de capture de chaque groupe de zones 7 sont en particulier choisis pour la mise en oeuvre de tests immunologiques au format sandwich et/ou au format compétition.

La présence de zone(s) additionnelle(s) de libération 6 permet ainsi une répartition optimale du ou des réactifs de détection au sein de chaque groupe de zones 7, qui peuvent alors être agencées à façon par rapport aux différentes zones de capture 5 associées.

Le réactif de détection au sein de chaque zone de libération 4, 6 peut ainsi être ajusté, en quantité et en spécificité, par rapport à la ou aux zones de capture 5 complémentaires, situées directement en aval.

De manière générale, le dispositif selon la présente invention permet la détermination d'analyte(s) aussi bien faiblement concentré(s), que très fortement concentré(s), dans un échantillon liquide sans obtenir de résultats faux positifs ou faux négatifs.

En outre, le dispositif selon la présente invention peut être structuré pour le dosage semi-quantitatif d'un analyte ou de plusieurs analytes dans un échantillon liquide.

Le dispositif selon l'invention est particulièrement adapté au dosage d'analytes présentant un effet crochet important comme l'hormone de grossesse (hCG), l'antigène prostatique spécifique (PSA ou « Prostate-Specific Antigen ») et l'hémoglobine (par exemple pour une détection d'hémoglobine dans les selles, dit encore test « FOB » pour « Fecal Occult Blood »).

Les autres avantages de l'invention, liés à la présence de plusieurs zones de libération, sont notamment :

- une optimisation des réactifs de détection et des réactifs de capture en fonction des concentrations cibles à détecter,
- un degré de précision plus élevé.

En conséquence des deux points ci-dessus, il est obtenu une augmentation de la sensibilité et de la spécificité du dispositif de détermination, ainsi que de la valeur d'interprétation du signal visible et mesurable.

Les différents aspects de la présente invention sont présentés plus en détails ci-dessous.

Analyte(s) et échantillon liquide

Par « analyte », on entend toute entité chimique, biochimique, ou biologique, que l'on souhaite détecter dans un échantillon.

Cette entité chimique consiste avantageusement en une entité issue du monde vivant, de préférence du monde végétal ou du monde animal, de préférence encore présent chez l'être humain.

Parmi les analytes détectés par les dispositifs et les procédés selon la présente invention, on citera notamment les protéines, les peptides, les anticorps, les hormones, les stéroïdes, les antigènes dérivés d'agents infectieux ou de cellules tumorales, les agents infectieux tels que les bactéries, les virus ou les parasites, les acides nucléiques (ADN ou ARN), les composés thérapeutiques, les drogues ou encore les antibiotiques.

L'analyte est en particulier choisi parmi ceux connus pour générer un effet crochet lors de leur détection par technique immunochromatographique.

Par « effet crochet » (ou « Hook effect » en anglais), on entend en particulier un résultat faux négatif obtenu par technique immunochromatographique, concluant de façon aberrante à l'absence de l'analyte dans l'échantillon, survenant lorsque l'analyte est présent dans l'échantillon à une concentration très élevée.

Généralement l'effet crochet produit un affaiblissement du signal réponse, visible et mesurable, inverse à l'augmentation de la concentration en analyte à déterminer, conduisant (i) à un signal identique (de même intensité) pour deux concentrations différentes, l'une faible et l'autre forte, et (ii) une inhibition de la révélation du signal pour une concentration en analyte extrêmement forte.

Par exemple, dans la plupart des tests immunochromatographiques dédiés à l'hormone hCG, l'effet crochet commence aux alentours de 5 000 mIU/mL et continuera jusqu'à environ 250 000 mIU/mL, impliquant une intensité de signal identique notamment pour une concentration faible (25mIU/mL) et forte (200 000 mIU/mL).

A cet égard, l'analyte est ainsi choisi avantageusement parmi l'hormone chorionique gonadotrope (ou « hCG »), l'antigène prostatique spécifique (PSA ou « Prostate-Specific Antigen »), l'hémoglobine, le FOB (« Fecal occult blood »), les marqueurs oncogéniques (tels que ferritine, AFP (alfa feto-protein), CA15-3/CA27.29 (cancer du sein), CA19-9 (cancer pancréatique), CA-125 (cancer ovarien)), la protéine C-réactive (« CRP » ou « C Reactive Protein »), la troponine I (« TNI » ou « Troponin I »), des marqueurs cardiaques (tels que troponine T, CK-MB, myoglobine, B-type natriuretic Peptide (t-BNP)), les DOA (pour « drugs of abuse »), les biomarqueurs pour le monitorage de thérapie (« Therapy monitoring biomarkers ») (TnF-alfa (tumor necrosis factor), autres thérapies associées avec des biomarqueurs oncogéniques circulants, autres biomarqueurs cellulaires, intracellulaires ou tissues spécifiques, etc.), l'hormone lutéinisante (« LH ») ou l'hormone folliculostimulante (« FSH »).

En fonction de leur structure, les dispositifs selon la présente invention permettent la détermination d'un analyte unique (mono-analyte) ou la détermination de plusieurs analytes (poly-, ou multi- ou pluri-analytes).

Par « plusieurs analytes », on entend au moins deux analytes, de préférence encore 2, 3, 4 ou 5 analytes.

Cette approche multi-analytes peut être intéressante pour l'étude des maladies auto-immunes (« Auto immune disease panel »), pour l'étude des allergies (« Allergies panel »), pour le monitorage des thérapies (« therapy monitoring ») dans le domaine du médicaments, de la toxicologie, de la réponse du patient à un traitement ou des marqueurs inflammatoires, pour les tests de diagnostic d'infection multiple (par exemple virus de l'immunodéficience humaine ou VIH/hépatite C/hépatite B).

Par exemple, le dispositif selon l'invention peut être adapté pour la détermination de différents anticorps dans un même échantillon liquide, à savoir par exemple anti-VIH (virus de l'immunodéficience humaine ou HIV), anti-HCV (virus de l'hépatite C), anti-HBs (marqueur de l'hépatite virale chronique), anti-TB (tuberculose).

Par « échantillon liquide », on entend tout échantillon dans lequel l'analyte recherché est en solution ou en suspension.

Cet échantillon liquide peut notamment être tout fluide biologique ou corporel.

L'échantillon liquide peut également avoir été obtenu directement ou indirectement à partir d'un fluide biologique ou corporel.

L'échantillon peut également être un extrait liquide d'un échantillon solide.

Typiquement, l'échantillon liquide est de l'urine, du sang total, du plasma ou du sérum.

Dans certains procédés selon la présente invention, un diluant est utilisé lorsque l'échantillon liquide est du plasma, du sérum ou du sang total par exemple.

Le diluant est déposé sur le support solide poreux avec l'échantillon. Alternativement, le diluant est déposé sur le support solide poreux avant ou après l'échantillon.

Ce diluant migre dans le support solide, entraînant, ou facilitant la migration de l'échantillon dans le support poreux, avec le réactif de détection marqué.

Typiquement ce diluant est composé d'une solution saline tamponnée, il peut également comprendre un détergent ou tout autre composant nécessaire à la réaction.

Le dispositif selon l'invention est intéressant en ce qu'il peut être adapté aussi bien à la détermination de la présence d'au moins un analyte, qu'à la détermination de sa quantité.

Par « détecter » ou « déterminer », on entend ainsi la détermination qualitative (avantageusement la présence ou l'absence) d'un ou plusieurs analytes dans un échantillon liquide.

Par « détecter » ou « déterminer », on entend aussi la mesure et la quantification d'un ou plusieurs analytes dans un échantillon.

En effet, les performances des dispositifs et procédés selon l'invention autorisent également des modes de réalisation pour la réalisation de mesures quantitatives ou semi-quantitatives d'un analyte ou d'au moins deux analytes différents dans un échantillon liquide.

Moyen de diffusion capillaire

Selon la présente invention, on entend par « moyen de diffusion capillaire 2 », tout moyen constituant ou agissant en tant qu'unité de diffusion capillaire continue, par migration latérale (c'est-à-dire perpendiculairement à l'épaisseur du ou des matériaux capillaires mis en oeuvre pour la diffusion capillaire).

Ce moyen de diffusion capillaire consiste avantageusement en un support solide poreux permettant la migration d'un liquide par simple diffusion capillaire.

La porosité de ce support permet la diffusion capillaire (ou migration latérale) de l'échantillon et/ou des réactifs à l'état liquide ou humide.

De tels moyens de diffusion capillaire sont très largement utilisés dans toutes les techniques d'immunochromatographie à migration latérale notamment.

Un tel moyen de diffusion capillaire est par exemple un support allongé selon la direction et/ou le sens de la diffusion capillaire (migration latérale).

Ce moyen de diffusion capillaire est constitué par :

- un seul et même matériau capillaire ou poreux, ou
- plusieurs éléments ou matériaux capillaires ou poreux différents, convenablement agencés les uns par rapport aux autres (par exemple par chevauchement), pour obtenir une continuité d'écoulement capillaire d'un élément ou d'un matériau à un autre, selon la direction de diffusion capillaire.

Un tel moyen de diffusion capillaire détermine une direction et sens de diffusion capillaire de tout liquide qui est reçu ou déposé à une extrémité amont, et qui se déplace alors vers une extrémité aval dudit moyen.

La diffusion capillaire considérée selon la présente invention, dite encore « immunochromatographique par migration latérale », est à distinguer de celle mise en oeuvre dans les techniques d'immuno-filtration, selon lesquelles les liquides cheminent dans l'épaisseur du ou des matériaux de filtration, poreux.

A titre d'exemple, ces moyens de diffusion capillaire peuvent être constitués de divers supports immunochromatographiques, par exemple de cellulose, de nylon, de nitrocellulose, de polyéthylène ou de fibre de verre.

Le moyen de diffusion capillaire peut être constitué d'une ou de plusieurs parties distinctes. Les différentes parties du support peuvent être constituées de matériaux différents. Lorsque le moyen de diffusion capillaire est constitué de différentes parties ou de différents matériaux, ces éléments sont disposés de telle façon à permettre la continuité de l'écoulement capillaire dans le moyen de diffusion capillaire.

Typiquement, le moyen de diffusion capillaire est constitué d'un support solide poreux allongé selon la direction de diffusion capillaire. De préférence, le moyen de diffusion capillaire des dispositifs selon l'invention comprend un support solide poreux en forme de bandelette immunochromatographique.

Le moyen de diffusion capillaire se présente par exemple sous la forme d'une bandelette immunochromatographique constituée de plusieurs bandelettes superposées ou chevauchantes.

Le dispositif selon l'invention peut par exemple être constitué d'une bandelette chromatographique fixée sur un support rigide.

Le support rigide peut être constitué de matériaux divers tels que du carton, du carton plastifié ou plus préférentiellement de matières plastiques. De préférence, le support rigide est constitué de polystyrène.

Avantageusement, le moyen de diffusion capillaire peut être incorporé dans un support de préhension. Ce support de préhension facilite la manipulation du moyen de diffusion capillaire, et peut également protéger celui-ci notamment de l'humidité.

Le support de préhension peut envelopper partiellement ou totalement le moyen de diffusion capillaire.

Le support de préhension peut être constitué de matériaux divers tels que du carton, du carton plastifié ou plus préférentiellement de matières plastiques. De façon avantageuse, le support de préhension est constitué d'un matériau rigide et imperméable.

Ces supports de préhension ou boîtiers sont notamment décrits dans les brevets WO-2007/023372, EP-0 291 194, EP-0 560 411, EP-0 560 410 et EP-1 091 808.

Habituellement, le support de préhension est en forme de boîtier.

Ce support de préhension est avantageusement pourvu d'au moins une fenêtre d'observation permettant d'observer les zones de capture.

Ces fenêtres d'observation sont avantageusement agencées de sorte à offrir un accès visuel direct uniquement aux zones de capture à analyser pour la détermination d'un analyte, ou le cas échéant pour la détermination d'au moins deux analytes.

Zone de dépôt

La zone de dépôt 3 correspond à une zone amont du moyen de diffusion capillaire 2 sur laquelle est rapporté l'échantillon liquide.

Cette zone de dépôt peut coopérer avec un organe de captation réalisé dans un matériau absorbant.

Cet organe de captation peut être directement mis en contact avec un flux d'urine par exemple.

Comme décrit dans WO-00/00288, l'organe de captation peut être mobile entre deux positions, l'une de recueil de l'échantillon liquide, à l'écart du moyen de diffusion capillaire, et l'autre en continuité ou en contact capillaire avec la zone de dépôt du moyen de diffusion capillaire.

Dans un mode de réalisation de l'invention, le moyen de diffusion capillaire peut comprendre une zone de dépôt ou recueil de l'échantillon, saillante par rapport au support de préhension, pour la réception de l'échantillon liquide.

Dans un autre mode de réalisation de l'invention, le support de préhension ou le boîtier comprend au moins une ouverture pour le dépôt de l'échantillon liquide.

Réactif de détection et réactif de capture

Sur sa longueur, le moyen de diffusion capillaire 2 comporte, d'une part, au moins un réactif de détection réparti pour matérialiser les zones de libération 4, 6 et, d'autre part, au moins un réactif de capture réparti pour matérialiser les zones de capture 5.

Par « zone de libération » ou « zone de capture » décrites plus en détails par la suite, on entend une partie localisée et délimitée du moyen de diffusion capillaire 2 sur lequel a été déposé une quantité d'au moins un réactif de détection ou d'au moins réactif de capture, respectivement.

Chacune des zones de libération 4, 6 et zones de capture 5 consiste avantageusement en une ligne ou bande transversale (s'étendant perpendiculairement à la direction de migration), présentant par exemple une largeur comprise entre 1 et 2 mm, et une surface comprise entre 3 et 5 mm$^2$.

De manière générale, le « réactif de détection » ou le « réactif de capture » consiste en toute entité chimique, biochimique ou biologique, qui est apte à se lier spécifiquement pour former un complexe permettant la détermination dudit analyte dans l'échantillon liquide.

Le réactif de détection et/ou le réactif de capture constituent encore des réactifs dits de « liaison ».

De tels réactifs de liaison, permettant la détermination de l'analyte ou de plusieurs analytes dans l'échantillon liquide, sont bien connus et peuvent être choisis à façon pour la mise en oeuvre de l'invention.

Ces réactifs de liaison sont choisis avantageusement parmi ceux qui sont aptes à se lier spécifiquement avec ledit analyte et/ou à se lier spécifiquement l'un avec l'autre.

Selon le format de test mis en oeuvre, les réactifs de liaison complémentaires sont destinés à former des complexes différents :

- les réactifs de liaison sont aptes à se fixer concomitamment à l'analyte, pour former un test au format sandwich,
- l'un des réactifs de liaison (détection ou capture) est apte à se fixer à l'analyte mais aussi à l'autre réactif de liaison (respectivement capture ou détection), pour former un test au format compétition.

Dans ce cadre, l'un au moins des réactifs de liaison est avantageusement choisi parmi les entités chimiques, biochimiques ou biologiques, aptes à se lier spécifiquement avec l'analyte et/ou à se lier spécifiquement avec un analogue de l'analyte.

Par « lier » ou « liaison », on entend toute liaison forte, par exemple covalente, mais aussi toute liaison faible, par exemple du type antigène/anticorps ou analyte / anti-analyte.

Par « anti-analyte », on entend toute entité chimique, biochimique, ou biologique, susceptible de se lier spécifiquement avec l'analyte, ou avec le réactif de capture en compétition avec l'analyte, par exemple un anticorps, un antigène ou un acide nucléique.

Par « analogue approprié de l'analyte », on entend avantageusement toute entité chimique, biochimique ou biologique, apte à se lier de manière spécifique au réactif de capture ou au réactif de détection, selon le cas, en compétition avec l'analyte.

Les réactifs de liaison sont avantageusement choisis parmi les anticorps, les antigènes ou les acides nucléiques.

L'analyte et le réactif de liaison forment ainsi typiquement un couple apte à se lier spécifiquement l'un avec l'autre, comme par exemple un coupe ligand/anti-ligand, un couple antigène/anticorps, un couple ADN/ARN ou un couple ADN/ADN.

Ainsi, si l'analyte est un antigène ou un haptène, l'un au moins des réactifs de liaison (le réactif de détection et/ou le réactif de capture) est avantageusement un anticorps spécifique de l'analyte.

Par « anticorps spécifique de l'analyte », on entend un anticorps capable de se lier spécifiquement avec l'analyte dans une liaison de type antigène/anticorps.

Il s'agit typiquement d'un anticorps polyclonal ou d'un anticorps monoclonal, ayant une forte affinité pour l'analyte. De préférence, il s'agit d'un anticorps monoclonal.

Si l'analyte est un anticorps, l'un au moins des réactifs de liaison est avantageusement l'antigène reconnu par l'anticorps.

Si l'analyte est un acide nucléique, l'un au moins des réactifs de liaison est avantageusement une sonde ADN complémentaire.

Le ou les réactifs de détection sont avantageusement conjugués à un marqueur visible et/ou mesurable, avantageusement un marqueur particulaire.

Par « marqueur visible et/ou mesurable », on entend tout marquage permettant une détection directe ou indirecte à l'oeil nu, ou à l'aide d'un appareil, en raison de l'émission d'un signal au niveau des zones de capture.

Le signal est par exemple une fluorescence, une coloration, une présence d'isotope ou un signal magnétique.

On citera par exemple les marqueurs particulaires colorés comme l'or colloïdal, ou fluorescents, les particules de latex colorées, les particules de latex fluorescentes et les particules conjuguées à l'avidine et à la streptavidine.

Les marqueurs particulaires, colorés ou fluorescents, consistent ainsi en des particules de petite taille insolubles dans l'eau et qui forment donc des suspensions, dispersions ou solutions, en phase liquide.

Parmi les marqueurs permettant une observation directe à l'oeil nu, on citera aussi les marqueurs de type dextran (Hansen T.M., IVD Technology 4, 35-40, 2003). Le réactif de liaison est alors conjugué à une chaîne de dextran (dérivé de polysaccharide) portant des fluorophores.

Les marqueurs peuvent également consister en des enzymes (la phosphatase alcaline ou AP, la peroxydase de raifort ou HRP, notamment), en des colorants (ou « dyes ») ou en des composés chimiluminescents (notamment l'isothiocyanate de fluorescéine ou FITC).

Pour augmenter la sensibilité, on peut avoir recours par exemple, à un anticorps marqué selon des techniques connues de l'homme de l'art pour une détection indirecte, comme par exemple un anticorps biotinylé, permettant indirectement une détection par la formation des entités avidine-biotine et streptavidine-biotine.

Cet anticorps marqué et biotinylé peut également, soit être directement déjà déposé sur une ligne-test, dans la zone de capture, pour augmenter la sensibilité, soit être déposé avec l'anticorps de détection spécifique, pour augmenter le temps de contact et encore la sensibilité notamment, par exemple, en raison du nombre de sites de fixation.

De son côté, le réactif de capture spécifique de l'analyte est immobilisé sur le support solide selon des techniques connues de l'homme du métier.

Ce réactif de capture est immobilisé de telle façon qu'il ne soit pas mobile à l'état humide.

Cette immobilisation peut s'effectuer par exemple par absorption ou par un couplage covalent.

Composition des groupes de zones successifs

Le moyen de diffusion capillaire 2 comporte ainsi une ou plusieurs zones de capture 5 qui sont complémentaires d'une zone de libération 4, 6 située directement en amont (tenant compte du sens de migration capillaire).

Par « directement en amont », on entend en particulier la première zone de libération 4, 6 qui se situe en amont de la zone de capture 5.

Par « complémentaire », on entend les zones de libération et de capture, constitutives d'un groupe de zones, dont les réactifs de liaison constitutifs sont choisis de sorte à permettre la mise en oeuvre d'un test pour la détermination de l'analyte d'intérêt, avantageusement un test au format compétition et/ou un test au format sandwich.

Chaque groupe de zones comprend ainsi une zone de libération suivie d'au moins une zone de capture complémentaire (le cas échéant avant la zone de libération d'un nouveau groupe de zones).

Tel qu'abordé précédemment, cet agencement a en particulier l'intérêt de permettre une adaptation au plus près et au plus juste, en quantité et/ou en spécificité, du réactif de détection d'une zone de libération en fonction du réactif de capture constituant la ou les zones de capture situées directement en aval.

De manière générale, le moyen de diffusion capillaire 2 peut comporter :

- exclusivement des groupes de zones dont les réactifs de liaison constitutifs sont choisis de sorte à permettre la mise en oeuvre de test au format compétition ; ou

- exclusivement des groupes de zones dont les réactifs de liaison constitutifs sont choisis de sorte à permettre la mise en oeuvre de test au format sandwich ; ou
- au moins un groupe de zones dont les réactifs de liaison constitutifs sont choisis de sorte à permettre la mise en oeuvre de test au format compétition, et au moins un groupe de zones dont les réactifs de liaison constitutifs sont choisis de sorte à permettre la mise en oeuvre de test au format sandwich ; ou
- au moins un groupe de zones dont les réactifs de liaison constitutifs sont choisis de sorte à permettre la mise en oeuvre de tests au format compétition et au format sandwich, et au moins un groupe de zones dont les réactifs de liaison constitutifs sont choisis de sorte à permettre la mise en oeuvre de test au format compétition et/ou au format sandwich.

Dans chaque groupe de zones, la zone de libération et la zone de capture, ou la première zone de capture d'une série, peuvent être confondues.

De manière alternative, la zone de capture, ou la première zone de capture d'une série, est séparée de la zone de libération selon une distance avantageusement de quelques millimètres, par exemple comprise entre 2 et 4 mm.

Les zones de capture d'une série sont en plus séparées les unes des autres selon une distance avantageusement de quelques millimètres, par exemple comprise entre 1 et 4 mm.

Encore de manière générale, la zone amont de libération peut être localisée dans la zone de dépôt ou réception de l'échantillon.

Mais, cette zone amont de libération peut aussi être déposée à l'état sec, en aval de la zone de dépôt ou réception de l'échantillon, pour éviter toute perte de réactif de détection par un effet de lavage lors du dépôt de l'échantillon.

Encore de manière générale, les zones de capture matérialisées sur le moyen de diffusion capillaire, complémentaires d'une zone de libération située directement en amont, sont avantageusement au nombre de 1 à 10, de préférence au nombre de 2 à 5 (c'est-à-dire encore un nombre choisi parmi 1, 2, 3, 4, 5, 6, 7, 8, 9, 10).

Une telle approche est en particulier utile pour l'obtention de résultats du type semi-quantitatif ou quantitatif.

En effet, tel qu'illustré dans les Exemples, le profil du signal obtenu sur les zones de capture successives peut être attribué à un domaine de concentration en analyte dans l'échantillon liquide. Tel qu'illustré dans les Exemples, le profil du signal obtenu sur les zones de capture successives peut également être attribué à une valeur précise de concentration en analyte dans l'échantillon liquide.

De même, la partie Exemple montre encore que, de manière surprenante, l'effet crochet peut être significativement réduit avec un agencement particulier des zones de libération et de capture adaptées pour la mise en oeuvre d'un test sandwich.

A cet égard, il est démontré qu'une forme de réalisation intéressante comprend :

- un groupe amont de zones comprenant le couple zone de libération / une zone de capture ou deux zones de capture, puis
- un groupe aval de zones comprenant au moins une nouvelle zone de libération suivie d'une série de zones de capture.

Ce groupe amont de zones est avantageusement basé sur un format sandwich, de manière à assurer la capture amont d'analyte(s) :

- soit une part significative d'analyte(s),
- soit une part correspondant à un seuil de détection ayant une valeur de diagnostic (la « valeur de diagnostic » se réfère à toute concentration en analyte (ou biomarqueur) scientifiquement reconnu comme seuil permettant une interprétation médicale).

Cette capture amont programmée permet notamment de contrôler la concentration dans l'échantillon liquide cheminant au sein du ou des groupes de zones postérieures.

La présence de ce groupe amont de zones présenterait l'intérêt de contrôler l'effet crochet dans le ou les groupes de zones en aval.

Toujours de manière générale, la concentration en réactif de détection au sein de la zone de libération et en réactif de capture au sein des zones de capture complémentaires est avantageusement ajustée notamment en fonction des fourchettes de concentration de valeur diagnostique recherchées, suivant la nomenclature de concentration exprimée en ng par mL ou en unité internationale (IU ou UI) par mL.

Cette concentration est par exemple comprise entre 0,1 et 1 ng / cm linéaire du support poreux, en fonction des spécificités des réactifs de liaison.

Par ailleurs, les zones de libération, et par conséquent les groupes de zones correspondants, sont au nombre de 1 à 4 (c'est-à-dire encore un nombre choisi parmi 1, 2, 3 ou 4). Le domaine préféré serait par exemple de deux groupes pour une longueur de support capillaire de 25 mm.

Le choix des réactifs de liaison pour les zones associées s'effectue à façon, de manière classique en soi.

Dans un test par compétition, les zones de libération 4, 6 et de capture 5 complémentaires comprennent avantageusement :

(i) un réactif de détection marqué sous la forme de l'analyte lui-même, ou un analogue approprié de l'analyte, conjugué à un marqueur visible et/ou mesurable, et
(ii) un réactif de capture immobilisé, avantageusement un anti-analyte du type anticorps, apte à se lier de façon spécifique à l'analyte présent dans l'échantillon et au réactif de détection précité.

Ce mode de réalisation permet la détection de l'analyte de l'échantillon liquide par un test dans lequel une compétition s'effectue au niveau de la ou des zones de capture complémentaires entre, d'une part, l'analyte de l'échantillon et, d'autre part, l'analyte marqué ou l'analogue d'analyte marqué.

De manière alternative, toujours dans un test par compétition, les zones de libération 4, 6 et de capture 5 complémentaires comprennent avantageusement :

(i) un réactif de détection marqué sous la forme d'un anti-analyte, par exemple un anticorps spécifique de l'analyte ou de l'analogue de l'analyte, conjugué à un marqueur visible et/ou mesurable, et
(ii) un réactif de capture sous la forme de l'analyte lui-même, ou un analogue de l'analyte, immobilisé dans l'une au moins des zones de capture 5 du moyen de diffusion capillaire 2.

Ce mode de réalisation permet la détection de l'analyte de l'échantillon liquide par un test de compétition dans lequel le réactif de détection est apte à se fixer, en compétition, soit avec l'analyte de l'échantillon soit avec le réactif de capture.

Dans ces différents tests par compétition, le complexe réactif de détection marqué / réactif de capture, immobilisé, se forme en absence de l'analyte d'intérêt dans l'échantillon. Ces complexes immobilisés génèrent ainsi un signal visible et/ou mesurable tel que défini ci-dessus, en l'absence d'analyte.

En présence d'analyte, il n'y a pas d'immobilisation du réactif de détection marqué au niveau du réactif de capture. L'absence de signal au niveau de la zone de capture correspond ainsi à la présence de l'analyte dans l'échantillon.

Dans un test de type sandwich, les zones de libération 4, 6 et de capture 5 complémentaires comprennent avantageusement :

(i) un réactif de détection marqué sous la forme d'un anti-analyte conjugué à un marqueur visible et/ou mesurable, se liant de façon spécifique à l'analyte, et
(ii) un réactif de capture immobilisé se liant de façon spécifique à l'analyte, avantageusement également un anti-analyte.

Selon une forme de réalisation préférée de ce mode de réalisation au format sandwich, l'une au moins des zones de libération 4, 6 d'un groupe de zones 7 comprend un ou des anticorps spécifiques de l'analyte ou d'au moins un analyte, conjugués à un marqueur visible et/ou mesurable, qui sont déposés à l'état sec dans ou sur le moyen de diffusion capillaire, mais qui sont libres de migrer par diffusion capillaire à l'état humide.

Dans ce cas, la ou les zones de capture 5 complémentaires de ce groupe de zones 7 comprennent un ou des anticorps spécifiques de l'analyte qui sont immobilisés selon des techniques connues. Ces anticorps sont immobilisés de telle façon qu'ils ne soient pas mobiles à l'état humide.

Les anticorps d'une zone de libération 4, 6 et de la ou des zones de capture complémentaires 5 se lient respectivement et spécifiquement avec l'analyte, par exemple sur deux sites épitopiques, identiques ou différents de l'analyte.

Les complexes analyte / anticorps obtenus sont immobilisés au niveau de la ou des zones de capture 5 du groupe de zones 7. Ces complexes immobilisés génèrent ainsi un signal visible et/ou mesurable tel que défini ci-dessus.

Pour la détection d'un unique analyte, le ou les réactifs de détection des zones de libération et/ou le ou les réactifs de capture des zones de capture sont avantageusement aptes à se lier spécifiquement avec ledit analyte.

Les groupes de zones successifs sont ainsi avantageusement dédiées à un même analyte.

Dans ce cas, les zones de libération comportent avantageusement un ou des réactifs de détection identiques entre elles, et les zones de capture comportent un ou des réactifs de capture identiques entre elles.

Les différents groupes de zones successifs sont ainsi tous adaptés pour la mise en oeuvre d'un test au même format, à savoir compétition ou sandwich.

De manière alternative, les zones de libération comportent avantageusement chacun un réactif de détection spécifique, et les zones de capture complémentaires comportent un réactif de capture complémentaire.

Dans ce cas, les différents groupes de zones successifs peuvent être adaptés pour la mise en oeuvre de tests identiques ou différents les uns des autres, à savoir compétition ou sandwich.

Pour la détection d'au moins deux analytes, le réactifs de détection d'une zone de libération et/ou le réactif de capture de la ou des zones de capture complémentaires, immédiatement en aval de ladite zone de libération et formant ensemble un groupe de zones, sont avantageusement aptes à se lier spécifiquement avec l'un desdits analytes à déterminer.

Dans ce cas, chaque groupe de zones est avantageusement dédié à l'un des analytes.

Les différents groupes de zones successifs peuvent être adaptés pour la mise en oeuvre de tests identiques (compétition ou sandwich) ou différents les uns des autres (compétition et sandwich).

Selon une forme intéressante de réalisation, chaque groupe de zone comprend une zone de capture finale, située en aval des zones de capture dudit groupe de zones.

Cette zone de capture finale comprend un réactif immobilisé qui est apte à se lier de façon spécifique au réactif de détection marqué provenant de la zone de libération dudit groupe de zone.

Cette forme de réalisation peut servir de zone de contrôle ; elle a également l'intérêt de permettre une capture du réactif de détection marqué en fin de ce groupe de zones, de sorte qu'il n'atteigne pas un groupe de zones situé en aval et afin de prévenir les éventuels phénomènes d'interférence.

Encore de manière générale, les zones de libération 4, 6 et de capture 5 complémentaires d'un groupe de zones 7 comprennent avantageusement :

(i) un réactif de détection marqué sous la forme d'un anti-analyte, par exemple un anticorps spécifique de l'analyte ou de l'analogue de l'analyte, conjugué à un marqueur visible et/ou mesurable,

(ii) un réactif de capture sous la forme de l'analyte lui-même, ou un analogue de l'analyte, immobilisé dans l'une au moins des zones de capture 5 du moyen de diffusion capillaire 2, et

(iii) un réactif de capture immobilisé se liant de façon spécifique à l'analyte, avantageusement également un anti-analyte, immobilisé dans une autre des zones de capture 5 du moyen de diffusion capillaire 2.

Cette dernière forme de réalisation permet la mise en oeuvre simultanée d'un test au format sandwich et d'un test au format compétition, au sein d'un même groupe de zones 7.

Modes de réalisation particuliers

Un premier mode de réalisation conforme à l'invention est illustré sur la figure 1.

Ce dispositif est adapté pour la détermination qualitative d'un analyte d'intérêt susceptible d'être présent dans l'échantillon liquide.

Le moyen de diffusion capillaire 2 comporte deux groupes de zones 7 :

- un groupe amont 71, se composant de la zone amont de libération 4 et d'une zone de capture amont 51, complémentaires l'une de l'autre, et
- un groupe aval 72, se composant de la zone aval de libération 6 et d'une zone de capture aval 52, complémentaires l'une de l'autre.

Dans chaque groupe de zones 7, les réactifs de liaison respectifs sont choisis pour la mise en oeuvre d'une détection de l'analyte d'intérêt par un test de compétition ou par un test sandwich tel que développé ci-dessus.

Ces deux groupes de zones 7 sont ainsi adaptés pour mettre en oeuvre:

- un même test de compétition ou un même test sandwich,
- un test de compétition pour l'un, et un test sandwich pour l'autre.

Dans tous les cas, la présence d'une zone de libération 4, 6 en amont de chaque zone de capture 5 permet d'adapter au plus juste la concentration dans le couple zone de libération / zone de capture.

La détermination d'un unique analyte peut encore être mise en oeuvre au moyen de différents dispositifs conformes à l'invention, ayant leurs avantages spécifiques.

Les figures 2 et 3 présentent de tels autres dispositifs, dont le moyen de diffusion capillaire 2 comporte un groupe de zones amont 71 comprenant :

- la zone amont de libération 4, et
- une première zone de capture 51, complémentaire de ladite zone amont de libération 4.

Selon le cas, deux variantes sont envisageables pour le ou les groupes de zones 72 situés directement en aval.

Dans une première variante selon la figure 2, le moyen de diffusion 2 comporte encore un groupe de zones aval 72 comprenant :

- une zone aval de libération 6, puis
- plusieurs secondes zones de capture 52 successives, ici au nombre de trois, complémentaires de ladite zone aval de libération 6.

Cette forme de réalisation présente l'intérêt d'offrir un résultat semi-quantitatif particulièrement fiable, s'affranchissant de l'effet crochet.

Selon cette forme de réalisation, pour la détermination de l'hormone hCG, il peut être envisagé un groupe de zones amont 71 dont la concentration et la spécificité des réactifs sont ajustées pour détecter uniquement la concentration de l'hormone hCG correspondant au premier jour d'absence de règles (« missed period »), c'est-à-dire aux alentours de 20 mIU/mL.

La zone aval de libération 6 contient un réactif de détection dont la concentration est plus élevée que celle présente au niveau de la zone amont de libération 4, permettant la détection et la distinction de différentes concentrations de l'analyte au niveau des secondes zones de captures 52 successives en aval.

Dans ce format, pour une concentration en hormone hCG correspondant au premier jour d'absence de règles (« missed period »), il est ainsi révélé un signal visuel et mesurable de détection uniquement au niveau de la première zone de capture 51.

Pour une concentration en hormone hCG correspondant à une ou plusieurs semaines de grossesse, le signal visuel et mesurable apparaîtra successivement au niveau des secondes zones de capture 52 en aval de la zone aval de libération 6, offrant une analyse semi-quantitative de la concentration en hormone hCG de l'échantillon étudié.

Selon le même format d'exécution, partant de réactifs adaptés à la détermination de l'antigène prostatique spécifique (ou PSA), il peut être détecté et distingué la « concentration seuil de valeur diagnostic » (4ng/mL), par rapport à d'autres concentrations plus élevées du même analyte (jusqu'à 200ng/mL), permettant là encore une approche semi-quantitative.

Encore selon le même format, au moyen de réactifs adaptés à la détermination de sang (hémoglobine) dans les selles (test FOB pour « Fecal Occult blood »), il peut être détecté et distingué la « concentration seuil de valeur diagnostic » (40ng/mL), par rapport à des concentrations plus élevées du même analyte (jusqu'à 1000ng/mL et plus), offrant ainsi un résultat semi-quantitatif.

Selon une alternative non représentée de la première variante selon la figure 2, le groupe de zones amont 71 comprend une seconde zone de capture, également complémentaire de la zone amont de libération 4.

Cette seconde zone de capture est située en aval de la zone amont de libération 4 et de la première zone de capture 51, mais toujours en amont du groupe de zones aval 72.

Dans ce cas, le moyen de diffusion capillaire 2 comporte une premier groupe de zones 7, amont, comprenant :

- la zone amont de libération 4,
- deux zones de capture amont 51 successives, complémentaires de ladite zone amont de libération 4,
  puis un second groupe de zones 7, aval, comprenant :
- la zone aval de libération 6, et
- plusieurs secondes zones de capture 52 successives, ici au nombre de trois, complémentaires de ladite zone aval de libération 6.

Dans une seconde variante selon la figure 3, le moyen de diffusion 2 comporte encore des groupes de zones aval 72 successifs, ici au nombre de trois, comprenant chacun :

- une zone de libération 6, et
- une zone de capture 52, complémentaire de ladite zone de libération 6 associée.

Dans le cas d'une détermination d'un seul analyte, cette forme de réalisation présente l'intérêt d'optimiser le seuil de détection dans les zones de libération et de capture successives.

Dans cette variante, il peut être intéressant d'augmenter progressivement la concentration en réactif de détection au sein des zones de libération 4, 6 successives de l'amont vers l'aval, tout en gardant une même concentration de réactif de capture dans chacune des zones de capture 52.

Cette approche a un intérêt dans le cadre d'une détection d'analyte ayant une valeur de diagnostic à différentes valeurs (seuils) de concentration (par exemple hCG, PSA ou hémoglobine).

Selon une autre approche de l'invention, les zones de libération et les zones de capture peuvent être adaptées pour la détermination de plusieurs analytes.

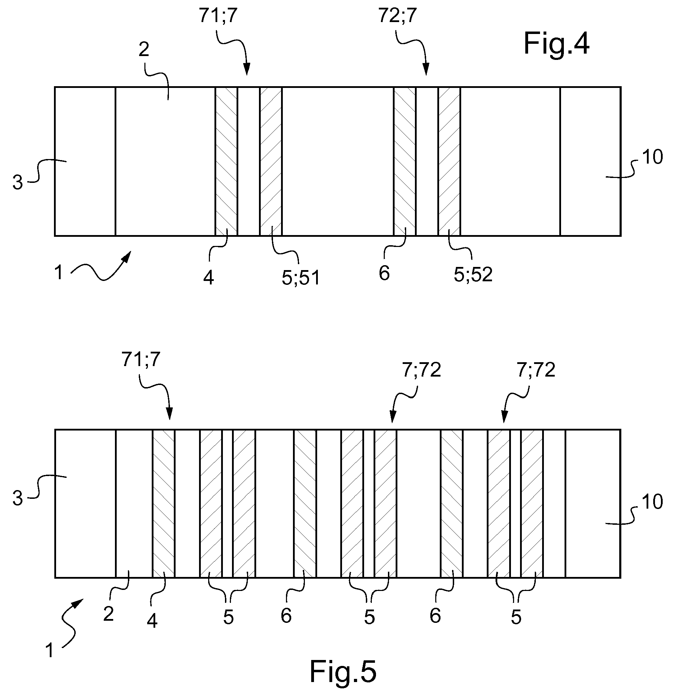

A cet égard, selon un mode de réalisation représenté sur la figure 4, le moyen de diffusion capillaire 2 comporte plusieurs groupes de zones 7 successifs, en l'occurrence au nombre de deux, composés chacun d'une zone de libération 4, 6 et d'une zone de capture complémentaire 5.

Dans ce cas, le groupe de zones amont 71 est adapté pour la détermination d'un premier analyte par un test approprié (compétition ou sandwich) ; le groupe de zones aval 72 est adapté pour la détermination d'un second analyte par un test approprié (compétition ou sandwich).

Un tel dispositif pourrait comporter encore un ou plusieurs autres groupes de zones 7 supplémentaires, adaptés chacun pour un analyte à déterminer.

De manière alternative, selon un mode de réalisation représenté sur la figure 5, le moyen de diffusion capillaire 2 comporte des groupes de zones 7, ici au nombre de trois, composés chacun d'une zone de libération 4, 6 suivie de plusieurs zones de capture complémentaires 5 (ici au nombre de deux).

Là encore, chaque groupe de zones 7 est adapté pour la détermination de l'un des analytes par un test approprié (compétition et/ou sandwich).

La présence de plusieurs zones de capture 5 au sein de chaque groupe de zones 7 permet l'obtention d'une valeur semi-quantitative pour chacun de ces analytes.

Ces différentes formes de réalisation ne sont aucunement limitatives. Les différents groupes de zones 7, et leurs compositions respectives, peuvent être adaptés à façon.

Zone témoin

Dans un mode de réalisation préféré de l'invention et tel qu'illustré sur les figures 1 à 5, le dispositif 1 comporte encore une zone témoin 10 qui est ménagée en aval et qui comprend un réactif de capture témoin.

Cette zone témoin 10 permet de disposer d'un contrôle positif afin de s'assurer de la diffusion capillaire effective de l'échantillon liquide depuis la zone de dépôt 3 jusqu'aux zones de capture 5 du moyen de diffusion capillaire 2.

Cette zone témoin 10 est constituée d'un réactif de capture qui est immobilisé de façon permanente en aval du moyen de diffusion capillaire 2.

Il peut s'agir par exemple d'un anticorps se liant au(x) réactif(s) de détection constitutif des zones de libération 4, 6. Dans ce cas, ce réactif de capture témoin permet de vérifier la migration du ou des réactifs de détection au travers des zones de capture 5.

Alternativement, ce réactif de capture témoin est indépendant de l'analyte et permet simplement de vérifier la diffusion de l'échantillon liquide le long du milieu de diffusion capillaire 2.

Mise en oeuvre

Un tel dispositif peut être mis en oeuvre selon le procédé ou mode d'emploi défini ci-après :

a) on dépose l'échantillon liquide dans la zone de dépôt 3 du moyen de diffusion capillaire 2,
b) on attend un temps suffisant pour la migration par diffusion capillaire de l'échantillon liquide jusqu'aux zones de capture 5, le cas échéant jusqu'à la zone témoin 10,
c) on observe au niveau des zones de capture 5 dans quelle mesure :

c.1) le réactif de détection, complexé avec l'analyte, se fixe au réactif de capture complémentaire de son groupe de zones 7 (format sandwich), et/ou
c.2) le réactif de détection se fixe au réactif de capture de son groupe de zones 7 (formant compétition), puis

d) on détermine qualitativement et/ou semi-quantitativement et/ou quantitativement l'analyte ou les analytes à partir des résultats obtenus.

En pratique, lors de la migration capillaire selon l'étape c), l'échantillon liquide traverse successivement chacun des groupes de zones 7 ménagés en série.

Dans chaque groupe de zones 7 mettant en oeuvre un test au format sandwich, les éventuels analytes présents forment un complexe avec les réactifs de détection issus de sa zone de libération 4 ou 6.

Les complexes ainsi formés cheminent jusqu'à la zone de capture complémentaire 5 où ils sont immobilisés par les réactifs de capture correspondants.

Les complexes ainsi retenus créent un signal visible et/ou mesurable au niveau de cette zone de capture, permettant la détermination de la présence de l'analyte d'intérêt dans ledit échantillon liquide.

Le cas échéant, l'intensité du signal augmente au niveau des zones de capture successives, réparties sur le ou les groupes de zones, jusqu'à épuisement progressif des complexes marqués.

L'intensité de signal est avantageusement mesurée au moyen d'un lecteur optique adapté. Cette intensité du signal est par exemple exprimée en nombre de pixels, détecté sur tout ou partie de la zone de capture 5.

Dans ce cadre, le dispositif selon l'invention est notamment intéressant lorsqu'il comporte plusieurs zones de capture successives pour un même analyte, destiné à pallier à l'effet crochet et pour l'obtention d'un résultat semi-quantitatif.

A cet égard, l'intensité du signal au niveau de chaque zone de capture est comparée par rapport à celle des autres zones de capture successives.

Une partie des complexes formés entre les réactifs de détection de la zone de libération et l'analyte est capturée par chaque zone de capture successives, jusqu'à épuisement progressif de l'analyte dans l'échantillon liquide en cours de migration.

L'intensité du signal au niveau de chaque zone de capture augmente ainsi par rapport à la zone de capture précédente, jusqu'à une zone de capture où cette intensité est maximale. Cette zone de capture est ensuite suivie de zones de capture où l'intensité du signal diminue.

Des solutions étalons sont mises en oeuvre préalablement sur un dispositif identique de manière à pouvoir attribuer un profil de résultat avec un résultat semi-quantitatif d'analyte dans l'échantillon liquide.

Il est alors possible de transformer ce résultat en une valeur semi-quantitative, par une attribution préalable de chaque zone de capture à un domaine de concentration de l'analyte dans un échantillon liquide.

Dans un groupe de zones 7 mettant en oeuvre un test au format compétition, les éventuels analytes présents empêchent la formation de complexes entre le réactif de détection issu de sa zone de libération 4 ou 6 et le réactif de capture de la ou des zones de captures complémentaires 5.

En l'absence d'analyte, les complexes se forment et créent un signal visible et/ou mesurable au niveau de la zone de capture correspondante.

Là encore, le dispositif selon l'invention est notamment intéressant lorsqu'il comporte plusieurs zones de capture successives pour un même analyte, destiné à pallier à l'effet crochet et pour l'obtention d'un résultat semi-quantitatif.

Les résultats peuvent être interprétés semi-quantitativement en fonction de la concentration en analyte, comme par exemple faible, moyen, fort et très fort.

Des solutions étalons sont mises en oeuvre préalablement sur un dispositif identique de manière à pouvoir là encore attribuer un profil de résultat avec un résultat semi-quantitatif d'analyte dans l'échantillon liquide.

Il est alors possible de transformer ce résultat en une valeur semi-quantitative, par une attribution préalable de chaque zone de capture à un domaine de concentration de l'analyte dans l'échantillon. De manière générale, le dispositif de détermination peut également permettre la détermination quantitative d'un analyte.

Le résultat visuel obtenu peut ainsi être transformé par exemple en une concentration, exprimée par exemple en ng par mL ou en mUI par mL de l'analyte dans l'échantillon liquide à analyser.

Pour cela, des solutions étalons sont mises en oeuvre sur le dispositif de détermination de manière à pouvoir attribuer un profil d'intensité avec une valeur quantitative d'analyte dans l'échantillon liquide.

Par exemple, l'intensité du signal (nombre de pixels) est mesurée au niveau de chaque zone de capture.

Pour chaque groupe de zones, les intensités du signal mesurées sur ses zones de capture sont ensuite additionnées.

Les intensités additionnées respectivement des différents groupes de zones sont transformées en un ratio correspondant aux intensités additionnées d'un groupe de zones par rapport aux intensités additionnées d'un autre groupe de zones.

Si nécessaire, ces ratios font ensuite l'objet d'une interpolation, par exemple une interpolation linéaire, associée avantageusement avec un coefficient de proportionnalité pour chaque intervalle prédéterminé de la concentration en analyte, pour l'obtention d'une courbe étalon correspondant au ratio d'intensité (en abscisse) en fonction de la concentration en analyte dans l'échantillon liquide (en ordonnée).

Il est alors possible d'associer un ratio d'intensité obtenu pour un échantillon analysé avec une valeur quantitative de l'analyte dans ledit échantillon liquide, tenant compte de la courbe étalon précitée.

Dans une forme de réalisation particulière, les réactifs de détection et de capture sont appliqués sur le support, de façon à obtenir :

- un groupe de zones amont comprenant deux zones de capture T1 et T2, après une 1ère zone de libération L1, et
- un groupe de zones aval comprenant trois zones de capture T3, T4 et T5, après la 2ème zone de libération L2.

Dans ce cas, le ratio d'intensité cumulatif s'apprécie avantageusement selon la formule (I) suivante :

Ratio d'intensité cumulatif = (Somme de l'intensité des trois zones de capture T3, T4 et T5 aval) / (Somme de l'intensité des deux zones de capture T1 et T2 amont)

Le ratio d'intensité cumulatif permet de construire une courbe du signal (intensité) en fonction de la concentration en analyte dans un échantillon liquide à analyser.

Une telle analyse quantitative est encore illustrée dans l'Exemple 2 ci-après.

## EXEMPLE

Exemple 1 : Dispositif immunochromatographique par flux latéral pour la détermination d'un analyte présentant un effet crochet

Une évaluation de l'effet crochet a été mise en oeuvre au travers de tests de détermination de valeurs de concentration semi-quantitative de hCG par immunochromatographie par flux latéral.

Pour mémoire, afin de combattre l'effet crochet, les dispositifs de détermination sont habituellement dotés d'un excès d'anticorps marqués au niveau de la zone de libération unique.

Par excès d'anticorps, on entend une concentration d'anticorps significativement supérieure à la concentration nécessaire pour déterminer le seuil d'intérêt diagnostique.

Différents dispositifs ont été construits à partir d'anticorps « hCG matched pairs antibodies » proposés par exemple par la société Fitzerald (anticorps mono/polyclonal).

Les anticorps anti-hCG ont été marqués avec de l'or colloïdal (Aldrich Sigma) selon la méthodologie standard (par exemple la technique développée par la société Organon Teknika).

Les anticorps anti-hCG ont été imprimés sur la membrane de nitrocellulose (NC) de la société Millipore selon différents agencements développés ci-après (Essais 1 à 5).

La concentration de ces anticorps déposée est ajustée de façon à obtenir une concentration de 0,5 à 1 $\mu$g/mL.

Les bandelettes de 4mm ont été testées avec différentes concentrations d'hormones hCG (Sigma) diluées dans de l'urine hCG négative. Les dilutions testées sont 0, 25, 50, 100, 1 000, 5 000, 50 000, 100 000, 250 000 et 500 000 mIU/mL.

Ces différentes concentrations correspondent chacune à un état d'avancement d'une grossesse dans des conditions normales (respectivement 0, 1, 2, 3, 4, 5, 6, 9 à 12, 13 à 16 semaines de grossesse).

Le protocole d'essai consiste en l'application de 150$\mu$L de chaque dilution sur la zone dépôt de la bandelette ; après 3 à 5 minutes, une observation de la présence et l'intensité de coloration est mise en oeuvre sur la ou les zones de capture de chaque bandelette.

Les résultats sont enregistrés et interprétés suivant les critères généralement adaptés pour la lecture de ce genre de tests qualitatifs, à savoir :

0 : absence de coloration
$\pm$ : légère coloration, douteux, « border-line », « ghost-line », difficilement visible
+ : coloration pâle mais clairement visible
++ : ligne clairement visible
+++ : coloration de ligne intense
++++ : coloration de ligne très intense

*Essai 1 : Dispositif avec une zone de libération unique et une zone de capture unique (ligne de test 1)*

Les résultats obtenus sont présentés dans le tableau n°1 ci-dessous.

**Tableau 1**

| | | Intensité de coloration | | | | | |
|---|---|---|---|---|---|---|---|
| | | Ligne de test 1 | | | | | |
| Semaine de grossesse | **Concentration HCG mIU/ml** | Ø | $\pm$ | + | ++ | +++ | ++++ |
| **0** | **0** | X | | | | | |
| **1** | **25** | | X | | | | |
| **2** | **50** | | | X | | | |

(suite)

| | | Intensité de coloration | | | | | |
| | | Ligne de test 1 | | | | | |
| Semaine de grossesse | Concentration HCG mIU/ml | Ø | ± | + | ++ | +++ | ++++ |
|---|---|---|---|---|---|---|---|
| 3 | 100 | | | | X | | |
| 4 | 1000 | | | | | X | |
| 5 | 5000 | | | | | X | |
| 6 | 50000 | | | | X | | |
| 9-12 | 100000 | | | X | | | |
| 13-16 | 250000 | | X | | | | |
| | 500000 | | X | | | | |

Le test rapide par immunochromatographie par flux latéral montre une linéarité de la courbe dose/réponse située entre 1000 et 5000 mIU/ml de hCG.

A partir de cette concentration maximale, la perte d'intensité du signal commence à se manifester à cause de l'effet crochet (« high dose hook effect »).

En conséquence, ce type d'essai ne permet pas de différentier, dans certaines intensités de coloration du résultat, si la concentration en hCG serait de 50 mIU/mL ou de 100 000 mIU/mL.

*Essai* 2: *Dispositif avec une zone de libération unique et deux zones de capture successives (ligne de test 1 et ligne de test 2)*

Des bandelettes contenant une deuxième ligne de capture (ligne de test 2) ont été produites, puis testées avec le même panel de concentration d'hCG.

Les résultats obtenus sont présentés dans le tableau n°2 ci-dessous.

**Tableau 2**

| | Intensité de coloration | | | | | | | | | | | |
| | Ligne de test 1 | | | | | | | Ligne de test 2 | | | | |
| Concentration HCG mIU/ml | Ø | ± | + | ++ | +++ | ++++ | | Ø | ± | + | ++ | +++ | ++++ |
|---|---|---|---|---|---|---|---|---|---|---|---|---|---|
| 0 | X | | | | | | | X | | | | | |
| 25 | | X | | | | | | X | | | | | |
| 50 | | | X | | | | | | X | | | | |
| 100 | | | | X | | | | | | X | | | |
| 1000 | | | | | X | | | | | | X | | |
| 5000 | | | | | X | | | | | | | X | |
| 50000 | | | | X | | | | | | | | X | |
| 100000 | | | X | | | | | | | | X | | |
| 250000 | | X | | | | | | | | | X | | |
| 500000 | | X | | | | | | | | | X | | |

L'effet crochet est reporté à une concentration supérieure. En effet, il apparaît que l'introduction d'une deuxième ligne de capture décale le seuil de concentration de l'effet crochet à une zone de concentration supérieure.

*Essai 3: Dispositif avec une zone de libération unique et trois zones de capture successives (ligne de test 1, ligne de test 2 et ligne de test 3)*

Des bandelettes contenant une troisième ligne de capture (ligne de test 3) ont été produites, puis testées avec le même panel de concentration d'hCG.

Les résultats obtenus sont présentés dans le tableau n°3 ci-dessous.

**Tableau 3**

| Semaine grossesse | Concentration HCG mIU/ml | Intensité de coloration | | | | | | | | | | | | | | | | | | |
|---|---|---|---|---|---|---|---|---|---|---|---|---|---|---|---|---|---|---|---|---|
| | | Ligne de test 1 | | | | | | Ligne de test 2 | | | | | | - | Ligne de test 3 | | | | | |
| | | Ø | ± | + | ++ | +++ | ++++ | Ø | ± | + | ++ | +++ | ++++ | | Ø | ± | + | ++ | +++ | ++++ |
| 0 | 0 | X | | | | | | X | | | | | | | X | | | | | |
| 1 | 25 | | X | | | | | X | | | | | | | X | | | | | |
| 2 | 50 | | | X | | | | | X | | | | | | X | | | | | |
| 3 | 100 | | | | X | | | | X | | | | | | | X | | | | |
| 4 | 1000 | | | | | X | | | | X | | | | | | X | | | | |
| 5 | 5000 | | | | | X | | | | | X | | | | | | X | | | |
| 6 | 50000 | | | | X | | | | | | X | | | | | | X | | | |
| 9-12 | 100000 | | | X | | | | | | X | | | | | | X | | | | |
| 13-16 | 250000 | | X | | | | | | X | | | | | | | X | | | | |
| | 500000 | | X | | | | | | X | | | | | | X | | | | | |

EP 2 828 661 B1

La troisième ligne de capture (ligne de test 3) ne déplace plus l'effet crochet. Cela est probablement dû à un épuisement des anticorps marqués de la zone de libération.

*Essai 4*: *Dispositif avec une zone de libération unique et trois zones de capture successives*

Suivant la conclusion de l'essai n°3, nous avons gardé les trois zones de capture tout en doublant la concentration des anticorps marqués de la zone de libération unique.
Cette configuration s'est révélée sujette à fort bruit de fond, rendant le test illisible et inexploitable.

*Essai 5* : *Dispositif avec deux zones de libération et quatre zones de capture*

Une deuxième zone de libération a été introduite après la première ligne de capture (ligne de test 1), succédée par 3 lignes complémentaires d'anticorps de capture. Cette configuration correspond à la forme de réalisation telle que décrite ci-dessus en relation avec la figure 2.
Cette nouvelle plateforme a été testée avec le même panel de concentrations de hCG. Les résultats obtenus sont récapitulés dans le tableau n°4 ci-dessous.
L'introduction d'une deuxième zone de libération, complémentée de 3 lignes de capture successives, permet d'affirmer dans quelle plage de concentration se situe le signal obtenu dans la première ligne de capture (ligne de test 1) et en conséquence se prononcer sur la fourchette de concentration se référant à l'état d'avancement de la grossesse de façon semi-quantitative.
Cette approche apporte une solution au problème d'effet crochet.

**Tableau 4**

| Semaine grossess | Concentration HCG mIU/ml | Intensité de coloration | | | | | | | | | | | | | | | | | | | | | | | |
|---|---|---|---|---|---|---|---|---|---|---|---|---|---|---|---|---|---|---|---|---|---|---|---|---|---|
| | | ZONE DE TEST 1 | | | | | | ZONE DE TEST 2: lignes de capture placées après la 2ème zone de libération | | | | | | | | | | | | | | | | | |
| | | Ligne de test 1 | | | | | | Ligne de test 2 | | | | | | Ligne de test 3 | | | | | | Ligne de test 4 | | | | | |
| | | Ø | ± | + | ++ | +++ | ++++ | Ø | ± | + | ++ | +++ | ++++ | Ø | ± | + | ++ | +++ | ++++ | Ø | ± | + | ++ | +++ | ++++ |
| 0 | 0 | X | | | | | | X | | | | | | X | | | | | | X | | | | | |
| 1 | 25 | | X | | | | | X | | | | | | X | | | | | | X | | | | | |
| 2 | 50 | | | X | | | | | X | | | | | X | | | | | | X | | | | | |
| 3 | 100 | | | | X | | | | | X | | | | | X | | | | | X | | | | | |
| 4 | 1000 | | | | | X | | | | | X | | | | | X | | | | | X | | | | |
| 5 | 5000 | | | | | X | | | | | | X | | | | | X | | | | | X | | | |
| 6 | 50000 | | | | X | | | | | | | | X | | | | | X | | | | | X | | |
| 9-12 | 100000 | | | X | | | | | | | | | X | | | | | | X | | | | | X | |
| 13-16 | 250000 | | X | | | | | | | | | X | | | | | | X | | | | | | | X |
| | 500000 | | X | | | | | | | X | | | | | | | | X | | | | | | X | |

Exemple 2: Dispositif immunochromatographique par flux latéral pour la détermination quantitative d'un analyte présentant un effet crochet

*Matériel*

Le dispositif immunochromatographique comprend un support poreux sous la forme d'une membrane nitrocellulose, préférablement avec une taille de pore entre 8 et 15 micromètres, et entre 2 et 3 cm de largeur.

Les matériaux poreux pour bandelette échantillon (« sample pad »), pour bandelette conjuguée (« conjugate pad ») et pour bandelette absorbante (« absorbent pad ») ont été sélectionnés parmi un choix multiple de fournisseurs de papiers en cellulose et en fibre de verre, avec une épaisseur entre 0,2 et 1,0 mm suivant l'application et une porosité entre 100 à 200 micromètres.

Les anticorps ont été construits à partir de « hCG matched pairs antibodies », proposés par la société BiosPacific (anticorps mono/polyclonal).

Les anticorps monoclonaux et polyclonaux anti-hCG et anti Beta-hCG sont préférablement à une concentration 1 mg/mL dans une solution tampon PBS.

Les anticorps anti-hCG ont été marqués avec de l'or colloïdal (Aldrich Sigma) selon la méthodologie standard (par exemple la technique développée par la société Organon Teknika).

Les anticorps anti-hCG ont été imprimés sur la membrane de nitrocellulose (NC) de la société Millipore.

La concentration de ces anticorps déposée est ajustée de façon à obtenir une concentration de 0,5 à 1 $\mu$g/mL.

L'or colloïdal pour préparation de réactifs de libération (marqueur) est préférablement à une concentration de 10 à 15 OD/mL, et de taille de particule entre 20 et 60 micromètres.

Une gamme de contrôles hCG (Sigma) a été préparée et calibrée (selon le standard international NIBSC), afin d'obtenir des concentrations d'hCG comprise entre 0 mIU/mL et 1 000 000 mIU/ml, nécessaires pour l'évaluation de performance du test et son contrôle qualité. Les dilutions testées sont 0, 25, 250, 2 500, 25 000, 250 000, 500 000 et 1 000 000 mIU/mL

*Méthode*

Les réactifs de détection et de capture sont appliqués sur le support en nitrocellulose, de façon à obtenir :

- une 1 ère zone de libération L1, avantageusement sur une bandelette conjuguée (« conjugate pad ») interposée entre une bandelette échantillon (« sample pad ») et une membrane imprimée sur support en PVC,
- deux zones amont de capture T1 et T2, après la 1ère zone de libération L1,
- une 2ème zone de libération L2, et
- trois zones aval de capture T3, T4 et T5, après la 2ème zone de libération L2.

La 2ème zone de libération L2 est agencée entre la seconde zone amont de capture T2 et la première zone aval de capture T3, de façon à assurer avantageusement une distance d'au minimum deux millimètres entre eux.

Les concentrations des réactifs des zones de libération et des zones de capture sont ajustées afin d'assurer la détection de l'analyte hCG recherché aux concentrations déterminés (0 à 1 000 000 mIU/mL).

Après l'application des réactifs, le support en nitrocellulose est séché dans des conditions de température et d'humidité programmées (généralement 1h à 37°C).

Après séchage, le support en nitrocellulose imprimé de réactifs est utilisé pour la construction d'un test au format « bande de test à couper en bandelette » (« mastersheet »), comprenant un support PVC, la membrane de nitrocellulose imprimée, un « conjugate pad » (1ère zone de libération), un « sample pad » et « absorbent pad ».

La bandelette échantillon (« sample pad »), la bandelette conjuguée (« conjugate pad ») et la bandelette absorbante (« absorbent pad ») sont des matériaux poreux qui sont agencées sur le support en PVC avec une membrane de nitrocellulose imprimée, adaptée et éventuellement traitée immuno-chimiquement, de façon à accueillir effectivement l'échantillon (« sample pad »), à le filtrer, à assurer sa réaction avec des réactifs dans la 1 ère zone de libération (« conjugate pad »), à assurer la migration uniforme sur le membrane et finalement à absorber l'excès de réactifs et à stopper la migration (« absorbent pad »).

La bande de test « mastersheet » est ensuite coupé pour obtenir des bandelettes de 2 à 6 mm de largeur, de préférence de 4mm ou 5mm de largeur.

Les bandelettes sont ensuite logées dans un boitier plastic de forme rectangulaire (dispositif format cassette), prêtes à être utilisées.

*Procédure de test*

Différentes concentrations de hCG sont préparées pour obtenir une gamme de contrôles couvrant des concentrations entre 0 et 1 millions mIU/mL d'hormone hCG.

Chaque concentration est testée en duplicata.

Généralement entre 100 et 200 microlitres d'échantillon de chaque concentration d'hCG sont appliqués sur les différentes unités de test.

La réaction est observée pendant une période de 10 minutes maximum.

Les résultats obtenus sont évalués à l'oeil nu (fourchette de concentration).

Puis, les résultats obtenus sont enregistrés par des moyens de lecture optique (instrument ou lecteur) permettant de mesurer l'intensité de chaque ligne de capture (exprimée en pixels).

Le résultat quantitatif (chiffré) apporté par le lecteur est calculé à partir d'un algorithme dont le calcul de base prend en compte le ratio d'intensité cumulatif selon la formule (I) suivante :

Ratio d'intensité cumulatif (RIC) = (Somme de l'intensité des trois zones de capture aval T3, T4 et T5) / (Somme de l'intensité des deux zones de capture amont T1 et T2)

<u>Formule (I)</u>

Les résultats obtenus dans les présentes conditions expérimentales sont présentés dans le tableau 5 ci-dessous.

Le ratio d'intensité cumulatif augmente avec l'augmentation de la concentration de l'analyte dans l'échantillon liquide.

Par interpolation, le ratio d'intensité cumulatif permet de construire une courbe du signal (intensité) en fonction de la concentration d'analyte dans l'échantillon liquide.

Il est ensuite possible de déterminer la concentration en hCG dans un échantillon liquide sur une gamme comprise entre 0 et 1 000 000 mIU/mL, tenant compte de cette courbe de signal et du ratio d'intensité cumulatif mesuré sur ledit dispositif de détermination en présence dudit échantillon liquide.

**Tableau 5**

| | **INTENSITE EN PIXELS / LIGNE DE CAPTURE** | | | | | | | |
|---|---|---|---|---|---|---|---|---|
| Concentration d'analyte hCG en mIU/mL | **0** | **25** | **250** | **2500** | **25000** | **250000** | **500000** | **1000000** |
| Ligne de Capture T1 | 18886 | 108928 | 234085 | 278087 | 176567 | 29346 | 16915 | 10131 |
| Ligne de Capture T2 | 17316 | 81398 | 173706 | 246662 | 146653 | 41915 | 24346 | 10415 |
| **Total 2 lignes** | 36202 | 190326 | 407791 | 524749 | 323220 | 71261 | 41261 | 20546 |
| | | | | | | | | |
| Ligne de Capture T3 | 6295 | 33339 | 113129 | 229576 | 142516 | 45046 | 28946 | 13308 |
| Ligne de Capture T4 | 1965 | 28398 | 111021 | 164484 | 164342 | 42531 | 34208 | 15331 |
| Ligne de Capture T5 | 3502 | 30833 | 61406 | 99659 | 121671 | 36346 | 38123 | 22162 |
| **Total 3 lignes** | 11762 | 92570 | 285556 | 493719 | 428529 | 123923 | 101277 | 50801 |
| | | | | | | | | |
| **Ratio Total Pixel (T3+T4+T5)/(T1 +T2)** | **0,325** | **0,486** | **0,700** | **0,941** | **1,326** | **1,739** | **2,455** | **2,473** |

**Revendications**

**1.** Dispositif pour la détermination de la présence et/ou de la quantité d'au moins un analyte susceptible d'être contenu dans un échantillon liquide, comportant un moyen de diffusion capillaire (2) sur lequel ledit échantillon liquide est destiné à migrer latéralement selon une direction et un sens de migration capillaire, et sur lequel sont matérialisées, dans ledit sens de migration capillaire amont vers aval, au moins :

- une zone (3) de dépôt de l'échantillon liquide,
- une zone amont (4) de libération qui comprend au moins un réactif de détection conjugué avec un marqueur visible et/ou mesurable, ledit réactif de détection étant apte à se déplacer en conséquence de la migration de l'échantillon liquide dans ledit moyen de diffusion capillaire, et
- au moins deux zones de capture (5) qui comprennent chacune au moins un réactif de capture, immobilisé sur ledit moyen de diffusion capillaire,

**caractérisé en ce que** ledit dispositif (1) comporte encore au moins une zone aval de libération (6) qui est matérialisée sur ledit moyen de diffusion capillaire (2) et qui se situe en aval de l'une au moins desdites zones de capture (5),

laquelle ou lesquelles zones aval de libération (6) sont chacune intercalées entre deux zones de capture (5),

laquelle zone aval de libération (6) comprend également au moins un réactif de détection conjugué à un marqueur visible et/ou mesurable, ledit réactif de détection étant apte à se déplacer en conséquence de la migration de l'échantillon liquide dans le moyen de diffusion capillaire (2), et

**en ce que** le réactif de détection d'une zone de libération (4, 6) et/ou le réactif de capture de la ou des zones de capture (5) complémentaires, situées directement en aval de ladite zone de libération (4, 6), sont aptes à se lier spécifiquement avec ledit analyte et/ou à se lier spécifiquement l'un avec l'autre, pour former un complexe permettant la détermination dudit analyte dans ledit échantillon liquide au niveau de ladite ou desdites zones de capture (5) complémentaires.

2.  Dispositif de détermination selon la revendication 1, **caractérisé en ce qu'**il comporte un groupe amont de zones (71) comprenant une zone de capture amont (51) située directement en aval de la zone de libération amont (4), lequel groupe amont de zones (71) est lui-même suivi d'un ou plusieurs groupes de zones (72) comprenant chacun une zone de libération (6) et une ou plusieurs zones de capture (5) complémentaires.

3.  Dispositif de détermination selon l'une quelconque des revendications 1 ou 2, pour la détermination d'un unique analyte, **caractérisé en ce que** le ou les réactifs de détection des zones de libération (4, 6) et/ou le ou les réactifs de capture des zones de capture (5) sont aptes à se lier spécifiquement avec ledit analyte.

4.  Dispositif de détermination selon la revendication 3, **caractérisé en ce que** les zones de libération (4, 6) comportent un ou des réactifs de détection identiques entre elles, et en que les zones de captures (5) comportent un ou des réactifs de capture identiques entre elles.

5.  Dispositif de détermination selon l'une quelconque des revendications 3 ou 4, **caractérisé en ce que** le moyen de diffusion capillaire (2) comporte :

- la zone amont de libération (4),
- une ou deux zones de capture amont (51), complémentaires de ladite zone amont de libération (4), puis
- une zone aval de libération (6), et
- au moins deux secondes zones de capture (52), complémentaires de ladite zone aval de libération (6).

6.  Dispositif de détermination selon l'une quelconque des revendications 3 ou 4, **caractérisé en ce que** le moyen de diffusion capillaire (2) comporte :

- la zone amont de libération (4),
- une ou deux zones de capture amont (51), complémentaires de ladite zone amont de libération (4), puis
au moins deux groupes de zones (72) successifs comprenant chacun :
- une zone de libération (6), et
- au moins une zone de capture (52), complémentaire de ladite zone de libération (6) associée.

7.  Dispositif de détermination selon l'une quelconque des revendications 1 ou 2, pour la détection d'au moins deux analytes, **caractérisé en ce que** le réactif de détection d'une zone de libération (4, 6) et/ou le réactif de capture de la ou des zones de capture (5) complémentaires, immédiatement en aval de ladite zone de libération (4, 6), sont aptes à se lier spécifiquement avec l'un desdits analytes.

8.  Dispositif de détermination selon l'une quelconque des revendications 1 à 7, **caractérisé en ce que** la ou les zones aval de libération (6) matérialisées sur le moyen de diffusion capillaire (2), chacune intercalée entre deux zones de capture (5), sont au nombre de 1 à 4.

9. Dispositif de détermination selon l'une quelconque des revendications 1 à 8, **caractérisé en ce que** la ou les zones de capture (5) matérialisées sur le moyen de diffusion capillaire (2), complémentaires de l'une des zones de libération (4, 6) située directement en amont, sont au nombre de 1 à 10.

10. Dispositif de détermination selon l'une quelconque des revendications 1 à 9, **caractérisé en ce que** le réactif de détection d'une zone de libération (4, 6) et le réactif de capture de la ou de l'une au moins des zones de capture complémentaires (5) sont aptes à se lier spécifiquement avec l'analyte ou l'un au moins des analytes, pour constituer un test au format sandwich.

11. Dispositif de détermination selon l'une quelconque des revendications 1 à 10, **caractérisé en ce que** le réactif de détection de l'une au moins des zones de libération (4, 6) et le réactif de capture de la ou de l'une au moins des zones de capture (5) complémentaires consistent, pour l'un, en un analogue de l'analyte à déterminer et, pour l'autre, en un réactif apte à se lier spécifiquement avec ledit analyte ou avec ledit analogue de l'analyte, pour constituer un test au format compétition.

12. Procédé pour la détermination quantitative d'un analyte dans un échantillon liquide déposé sur un dispositif de détermination selon l'une quelconque des revendications 3 à 6, **caractérisé en ce qu'**il comprend les étapes successives suivantes :

- une étape de mesure de l'intensité du signal au niveau de chaque zone de capture (51, 52),
- dans chaque groupe de zones (7), une étape d'additionnement desdites intensités mesurées,
- une étape de calcul d'un ratio d'intensité correspondant aux intensités additionnées pour un groupe de zones par rapport aux intensités additionnées pour un autre groupe de zones, et
- une étape d'association dudit ratio d'intensité calculé à une valeur quantitative de l'analyte dans ledit échantillon liquide partant d'une courbe étalon correspondant au ratio d'intensité calculé en fonction d'une quantité d'analyte dans ledit échantillon liquide.

**Patentansprüche**

1. Vorrichtung zur Bestimmung des Vorhandenseins und/oder der Menge von mindestens einem möglicherweise in einer Flüssigkeitsprobe enthaltenen Analyten, die ein Mittel zur kapillaren Diffusion (2) aufweist, auf dem sich die Flüssigkeitsprobe seitlich in einer Richtung und in einem kapillaren Bewegungssinn bewegt und auf dem, im besagen kapillaren Bewegungssinn von oberhalb nach unterhalb, wenigstens folgendes verwirklicht ist:

- eine Zone (3) zur Ablage der Flüssigkeitsprobe,
- eine oberhalb gelegene Zone (4) zur Freisetzung, die wenigstens ein mit einem sichtbaren und/oder meßbaren Marker versetztes Erfassungsreagenz aufweist, wobei das Erfassungsreagenz geeignet ist, sich als Folge der Bewegung der Flüssigkeitsprobe im Mittel zur kapillaren Diffusion zu bewegen, und
- wenigstens zwei Auffangzonen (5), von denen jede wenigstens ein auf dem Mittel zur kapillaren Diffusion festgelegtes Auffangreagenz aufweist,
dadurch gelcennzeichnet, daß die Vorrichtung (1) noch wenigstens eine unterhalb gelegene Zone (6) zur Freisetzung aufweist, die auf dem Mittel zur kapillaren Diffusion (2) verwirklicht ist und die unterhalb von wenigstens einer der Auffangzonen (5) liegt,
wobei die unterhalb gelegene Zone (6) oder die unterhalb gelegenen Zonen (6) zur Freisetzung zwischen zwei Auffangzonen (5) gesetzt sind,
wobei die unterhalb gelegene Zone zur Freisetzung (6) auch wenigstens ein Erfassungsreagenz aufweist, wobei das Erfassungsreagenz geeignet ist, sich als Folge der Bewegung der Flüssigkeitsprobe im Mittel zur kapillaren Diffusion (2) zu bewegen,
und
daß das Erfassungsreagenz einer Zone zur Freisetzung (4, 6) und/oder das Auffangreagenz der direkt unterhalb der Zone zur Freisetzung (4, 6) gelegenen Auffangzone oder den komplementären Auffangzonen (5) geeignet sind, sich spezifisch mit dem Analyten und/oder spezifisch untereinander zu verbinden, um einen Komplex zu bilden, der die Bestimmung des Analyten in der Flüssigkeitsprobe in der Auffangzone oder den komplementären Auffangzonen (5) ermöglicht.

**2.** Vorrichtung zur Bestimmung gemäß Anspruch 1, **dadurch gekennzeichnet, daß** sie eine oberhalb gelegene Gruppe von Zonen (71) aufweist, die eine oberhalb gelegene Auffangzone (51) aufweist, die direkt unterhalb der oberhalb gelegenen Zone zur Freisetzung (4) liegt, wobei die oberhalb liegende Gruppe von Zonen (71) selbst von einer oder mehreren Gruppen von Zonen (72) gefolgt ist, die jeweils eine Zone zur Freisetzung (6) und eine oder mehrere komplementäre Auffangzonen (5) aufweisen.

**3.** Vorrichtung zur Bestimmung gemäß einem der Ansprüche 1 oder 2 zur Bestimmung eines einzigen Analyten, **dadurch gekennzeichnet, daß** das oder die Erfassungsreagenzien der Zonen zur Freisetzung (4, 6) und/oder das oder die Auffangreagenzien der Auffangzonen (5) geeignet sind, sich spezifisch mit dem Analyten zu verbinden.

**4.** Vorrichtung zur Bestimmung gemäß Anspruch 3, **dadurch gekennzeichnet, daß** die Zonen zur Freisetzung (4, 6) ein oder mehrere untereinander identische Auffangreagenzien aufweisen.

**5.** Vorrichtung zur Bestimmung gemäß einem der Ansprüche 3 oder 4, **dadurch gekennzeichnet, daß** das Mittel zur kapillaren Diffusion (2)

- die oberhalb liegende Zone zur Freisetzung (4),
- eine oder zwei oberhalb liegende, zur oberhalb liegenden Zone zur Freisetzung (4) komplementäre Auffangzonen (51), dann
- eine unterhalb liegende Zone zur Freisetzung (6) und
- wenigstens zwei zweite, zur unterhalb liegenden Zone zur Freisetzung (6) komplementäre Auffangzonen (52) aufweist.

**6.** Vorrichtung zur Bestimmung gemäß einem der Ansprüche 3 oder 4, **dadurch gekennzeichnet, daß** das Mittel zur kapillaren Diffusion (2)

- die oberhalb liegende Zone zur Freisetzung (4),
- eine oder zwei oberhalb liegende, zur oberhalb liegenden Zone zur Freisetzung (4) komplementäre Auffangzonen (51), dann wenigstens zwei aufeinanderfolgende Gruppen von Zonen (72) aufweist, von denen jede
- eine Zone zur Freisetzung (6) und
- wenigstens eine zur zugehörigen Zone zur Freisetzung (6) komplementäre Auffangzone (52) aufweist.

**7.** Vorrichtung zur Bestimmung gemäß einem der Ansprüche 1 oder 2 zur Erfassung von wenigstens zwei Analyten, **dadurch gekennzeichnet, daß** das Erfassungsreagenz einer Zone zur Freisetzung (4, 6) und/oder das Auffangreagenz der Auffangzone oder der komplementären Auffangzonen (5), die direkt unterhalb der Zone zur Freisetzung (4, 6) liegen, geeignet sind, sich spezifisch mit einem der Analyten zu verbinden.

**8.** Vorrichtung zur Bestimmung gemäß einem der Ansprüche 1 bis 7, **dadurch gekennzeichnet, daß** die unterhalb liegende Zone bzw. liegenden Zonen zur Freisetzung (6), die auf dem Mittel zur kapillaren Diffusion (2) verwirklicht sind, wobei jede zwischen zwei Auffangzonen (5) gesetzt ist, zwischen 1 und 4 umfaßt.

**9.** Vorrichtung zur Bestimmung gemäß einem der Ansprüche 1 bis 8, **dadurch gekennzeichnet, daß** die auf dem Mittel zur kapillaren Diffusion (2) verwirklichte Auffangzone oder die verwirklichten Auffangzonen (5), die zu einer der direkt oberhalb liegenden Zonen zur Freisetzung (4, 6) komplementär sind, zwischen 1 und 10 umfaßt.

**10.** Vorrichtung zur Bestimmung gemäß einem der Ansprüche 1 bis 9, **dadurch gekennzeichnet, daß** das Erfassungsreagenz einer Zone zur Freisetzung (4, 6) und das Auffangreagenz der Auffangzone oder wenigstens einer der komplementären Auffangzonen (5) geeignet sind, sich spezifisch mit dem Analyten oder wenigstens einem der Analyten zu verbinden, um einen Test im Sandwichformat zu bilden.

**11.** Vorrichtung zur Bestimmung gemäß einem der Ansprüche 1 bis 10, **dadurch gekennzeichnet, daß** das Erfassungsreagenz einer Zone zur Freisetzung (4, 6) und das Auffangreagenz der Auffangzone oder wenigstens einer der komplementären Auffangzonen (5) zum einen aus einem zum zu bestimmenden Analyten Analogen und zum anderen aus einem Reagenz bestehen, das geeignet ist, sich spezifisch mit dem besagten Analyten oder dem besagten Analogen des Analyten zu verbinden, um einen Test im Wettbewerbsformat zu bilden.

**12.** Verfahren zur quantitativen Bestimmung eines Analyten in einer auf einer Vorrichtung zur Bestimmung gemäß einem der Ansprüche 3 bis 6 abgelegten Flüssigkeitsprobe, **dadurch gekennzeichnet, daß** es die folgenden aufeinanderfolgenden Schritte aufweist:

- einen Schritt zum Messen der Stärke des Signals im Bereich jeder Auffangzone (51, 52),
- in jeder Gruppe von Zonen (7) eine Schritt zum Addieren der gemessenen Stärken,
- einen Schritt zum Berechnen eines Stärkeverhältnisses, das den addierten Stärken für eine Gruppe von Zonen im Verhältnis zu den addierten Stärken für eine andere Gruppe von Zonen entspricht, und
- einen Schritt zum Zuordnen des berechneten Stärkeverhältnisses zu einem quantitativen Wert des Analyten in der Flüssigkeitsprobe, auf der Grundlage einer Eichkurve, die dem berechneten Stärkeverhältnis in Abhängigkeit von einer Menge Analyt in der Flüssigkeitsprobe entspricht.

## Claims

1. Device for the determination of the presence and/or the amount of at least one analyte capable of being contained in a liquid sample, comprising a capillary diffusion means (2) on which said liquid sample is intended to migrate laterally according to a direction and a sense of capillary migration, and on which are materialized, in said upstream to downstream capillary migration sense, at least:

- one zone (3) for depositing the liquid sample,
- one upstream release zone (4) which comprises at least one detection reagent conjugated to a visible and/or measurable marker, said detection reagent being capable to move as a consequence of the migration of the liquid sample in said capillary diffusion means, and
- at least two capture zones (5) which comprise each at least one capture reagent immobilized on said capillary diffusion means,

**characterized in that** said device (1) further comprises at least one downstream release zone (6) which is materialized on said capillary diffusion means (2) and which is located downstream of at least one of said capture zones (5),

wherein the downstream release zone(s) (6) are each inserted between two capture zones (5),

which downstream release zone (6) comprises also at least one detection reagent conjugated to a visible and/or measurable marker, said detection reagent being capable to move as a consequence of the migration of the liquid sample in the capillary diffusion means (2), and

**in that** the detection reagent of a release zone (4, 6) and/or the capture reagent of the complementary capture zone(s) (5), located directly downstream of said release zone (4, 6), are capable of specifically binding to said analyte and/or specifically binding to each other to form a complex allowing the determination of said analyte in said liquid sample at said complementary capture zone(s) (5).

2. Device for determination according claim 1, **characterized in that** it comprises an upstream group of zones (71) comprising an upstream capture zone (51) located directly downstream of the upstream release zone (4), which upstream group of zone (71) being itself followed by one or more zone group(s) (72) each comprising a release zone (6) and one or more complementary capture zones (5).

3. Device for determination according to any one of claims 1 or 2, for the determination of a single analyte, **characterized in that** the detection reagent(s) of the release zones (4, 6) and/or the capture reagent(s) of the capture zones (5) are capable of specifically binding to said analyte.

4. Device for determination according to claim 3, **characterized in that** the release zones (4, 6) include one or more detection reagents identical to each other, and **in that** the capture zones (5) include one or more capture reagents identical to each other.

5. Device for determination according to any one of claims 3 or 4, **characterized in that** the capillary diffusion means (2) includes:

- the upstream release zone (4),
- one or two upstream capture zone(s) (51), complementary to said upstream release zone (4), and then
- a downstream release zone (6), and
- at least two second capture zones (52), complementary to said downstream release zone (6).

6. Device for determination according to any one of claims 3 or 4, **characterized in that** the capillary diffusion means (2) includes:

- the upstream release zone (4),
- one or two upstream capture zone(s) (51), complementary to said upstream release zone (4), and then at least two successive zone groups (72) each comprising:
- a release zone (6), and
- at least one capture zone (52), complementary to said related release zone (6).

7. Device for determination according to any one of claims 1 or 2, for the detection of at least two analytes, **characterized in that** the detection reagent of a release zone (4, 6) and/or the capture reagent of the complementary capture zone(s), immediately downstream of said release zone (4, 6), are capable of specifically binding to one of said analytes.

8. Device for determination according to any one of claims 1 to 7, **characterized in that** the number of downstream release zone(s) (6) materialized on the capillary diffusion means (2), each inserted between two capture zones (5), is from 1 to 4.

9. Device for determination according to any one of claims 1 to 8, **characterized in that** the number of capture zone(s) (5) materialized on the capillary diffusion mean (2), complementary to one of the release zones (4, 6) located directly upstream, is from 1 to 10.

10. Device for determination according to any one of claims 1 to 9, **characterized in that** the detection reagent of a release zone (4, 6) and the capture reagent of the complementary capture zone (5) or of at least one of them are capable of specifically binding to the analyte or at least one of the analytes to constitute a sandwich format test.

11. Device for determination according to any one of claims 1 to 10, **characterized in that** the detection reagent of at least one of the release zones (4, 6) and the capture reagent of the complementary capture zone (5) or at least one of them consist, in one case, in an analogue of the analyte to be determined and, in the other case, in a reagent capable of specifically binding to said analyte or to said analogue of the analyte, to constitute a competition format test.

12. Process for the quantitative determination of an analyte in a liquid sample deposited on a device for determination according to any one of claims 3 to 6, **characterized in that** it comprises the following successive steps:

- a step of measuring the signal strength at each capture zone (51, 52),
- in each zone group (7), a step of adding said measured strengths,
- a step of calculating a strength ratio corresponding to the added strengths for one zone group relative to the added strengths for another zone group, and
- a step of associating said calculated strength ratio to a quantitative value of the analyte in said liquid sample from a standard curve corresponding to the strength ratio calculated on the basis of an analyte amount in said liquid sample.

Fig.1

Fig.2

Fig.3

Fig.4

Fig.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007023372 A
- EP 1657550 A
- EP 0291194 A
- EP 0560411 A
- EP 0560410 A
- EP 1091808 A
- WO 0000288 A

**Littérature non-brevet citée dans la description**

- **HANSEN T.M.** *IVD Technology,* 2003, vol. 4, 35-40